# EUROPEAN PATENT APPLICATION

(11) **EP 2 463 389 A1**
(43) Date of publication of application: **13.06.2012**
(21) Application number: 11190144.3
(22) Date of filing: 19.10.2007
(51) Int. Cl.: C12Q 1/70

(54) **Methodology for analysis of sequence variations within the HCV NS5B genomic region**

(30) Priority: 20.10.2006 US 852983 P; 20.10.2006 US 852982 P; 20.10.2006 US 852984 P; 20.10.2006 US 853015 P; 20.10.2006 US 853016 P; 20.10.2006 EP 06122668; 20.10.2006 EP 06122675; 20.10.2006 EP 06122670; 20.10.2006 EP 06122678; 20.10.2006 EP 06122673
(62) Divisional of application: 07821605.8
(71) Applicant: Innogenetics N.V., 9052 Gent (BE)
(72) Inventor: Sablon, Erwin, 1785 Merchtem (BE); Libbrecht, Evelien, 8940 Geluwe (BE)
(74) Representative: BiiP cvba

(57) **Abstract**

The current invention relates to a standardized method for amplification of an HCV NS5B nucleic acid fragment of any one of HCV genotypes 1 to 6 as a tool for analysis of sequence variations that may be correlated with HCV drug resistance.

## Description

### FIELD OF THE INVENTION

The invention relates to the field of sequence variations within the HCV genome. More specifically, the invention relates to methods for amplification of an NS5B nucleic acid of any one of HCV genotypes 1 to 6 as a tool for analysis of sequence variations within the amplified NS5B fragment that may be correlated with HCV drug resistance. In particular, the methods comprise the design of sense and antisense primers according to genotype within NS5B genomic regions. Further subject of the invention are sets of primers designed according to genotype and capable of annealing to said NS5B genomic regions. The current invention further relates to kits based on said methods and primers.

### BACKGROUND OF THE INVENTION

The about 9.6 kb single-stranded RNA genome of the HCV virus comprises a 5'- and 3'-non-coding region (NCRs) and, in between these NCRs, a single long open reading frame of about 9 kb encoding an HCV polyprotein of about 3000 amino acids.

HCV polypeptides are produced by translation from the open reading frame and cotranslational proteolytic processing. Structural proteins are derived from the amino-terminal one-fourth of the coding region and include the capsid or Core protein (about 21 kDa), the E1 envelope glycoprotein (about 35 kDa) and the E2 envelope glycoprotein (about 70 kDa, previously called NS1), and p7 (about 7kDa). The E2 protein can occur with or without a C-terminal fusion of the p7 protein (Shimotohno et al. 1995). Recently, an alternative open reading frame in the Core-region was found which is encoding and expressing a protein of about 17 kDa called F (Frameshift) protein (Xu et al. 2001); Ou & Xu in US Patent Application Publication No. US2002/0076415). In the same region, ORFs for other 14-17 kDa ARFPs (Alternative Reading Frame Proteins), A1 to A4, were discovered and antibodies to at least A1, A2 and A3 were detected in sera of chronically infected patients (Walewski et al. 2001). From the remainder of the HCV coding region, the non-structural HCV proteins are derived which include NS2 (about 23 kDa), NS3 (about 70 kDa), NS4A (about 8 kDa), NS4B (about 27 kDa), NS5A (about 58 kDa) and NS5B (about 68 kDa) (Grakoui et al. 1993).

HCV is the major cause of non-A, non-B hepatitis worldwide. Acute infection with HCV (20% of all acute hepatitis infections) frequently leads to chronic hepatitis (70% of all chronic hepatitis cases) and end-stage cirrhosis. It is estimated that up to 20% of HCV chronic carriers may develop cirrhosis over a time period of about 20 years and that of those with cirrhosis between 1 to 4%/year is at risk to develop liver carcinoma (Shiffman 1999; Lauer and Walker 2001). An option to increase the life-span of HCV-caused end-stage liver disease is liver transplantation (30% of all liver transplantations world-wide are due to HCV-infection).

It is now well established that HCV exists as distinct genotypes among different HCV isolates with prevalence of each of the genotypes in specific geographical locations. At present, HCV variants are primarily classified into 6 genotypes, representing the 6 genetic groups defmed by phylogenetic analysis of core/E1 and NS5B subgenomic sequences as well as of complete genome sequences. Within each genotype, HCV variants can be further divided into subtypes (Simmonds et al. 1994). An overview of the current unified system of nomenclature of HCV genotypes is given by Simmonds et al. (2005).

HCV has a high mutation rate, which is thought to help the virus in eluding its host's immune system. The high mutation rate is reflected in the presence of many distinct HCV genome sequences, known as quasispecies, within infected individuals (Bukh et al. 1995; Farci et al. 1997). Quasispecies result from the activity of the virally-encoded NS5B RNA-dependent RNA polymerase, which, because of its lack of proofreading function, is inherently a low-fidelity enzyme. The possible biological consequences of quasispecies include: (i) the development of escape mutants to humoral and cellular immunity leading to the establishment of a persistent infection; (ii) variable cell tropism (e.g., lymphotropic vs hepatotropic); (iii) vaccine failure, and (iv) rapid development of drug resistance.

With the current standard FDA approved antiviral therapy of pegylated interferon α in combination with ribavirin, only approximately half of genotype-1 HCV infected individuals eligible for this treatment achieve a sustained virological response (Manns et al. 2001). Because of severe side effects and other medical complications, up to 75% of the HCV patients are excluded from therapy today. Therefore, new anti-HCV drugs that are currently under development and in early stage clinical trials, are aimed at employing new targets such as the HCV internal ribosome entry site (IRES), the HCV NS3 serine protease and the HCV NS5B RNA-dependent RNA polymerase (see e.g. Tan et al. 2002; De Francesco et al. 2003). The compounds under clinical evaluation comprise polymerase inhibitors (ViroPharma/Wyeth; Idenix Pharmaceuticals; Roche; XTL) and protease inhibitors (Schering-Plough; Vertex Pharmaceuticals; Gilead/Achillion; Intermune).

However, the high mutation rate and variability of HCV are expected to favour the emergence of drug resistance, limiting the clinical usefulness of these inhibitors. In fact, drug resistance mutations have already been discovered during *in vitro* experiments and initial clinical trials. For example, HCV sub-genomic replicons have been used to study viral resistance to both nucleoside and non-nucleoside NS5B inhibitors as well as to NS3/4A protease inhibitors (Kukolj et al. 2005; Mo et al. 2005; reviewed in: Tomei et al. 2005). An understanding of HCV resistance mutants would further progress towards effective HCV treatments. In other words, evaluating the susceptibility of existing drug resistance mutants to different classes of antiviral agents is of utmost importance for the development of new drugs. In addition, exploring the effect of combination treatment on drug resistance may provide insight into future HCV treatment strategies. All this could enable appropriate therapeutic protocols to be rapidly developed and/or to be altered. As such, treating HCV-infected patients with an appropriate, *i.e.* active, drug would become much more feasible.

In order to perform such accurate drug resistance profiling across HCV genotypes, methods are needed to efficiently amplify and analyze HCV nucleic acid sequences.

### SUMMARY OF THE INVENTION

In a first aspect, the present invention relates to a method for amplification of an HCV NS5B nucleic acid of any one of HCV genotypes 1 to 6 comprising the step of designing a set of primers per genotype in the NS5B genomic region, wherein said set of primers for amplifying said HCV NS5B nucleic acid is characterized in that at least one of the primers is an antisense primer that is capable of annealing to the NS5B genomic region spanning nucleotides 9276 to 9298, wherein the nucleotide positions correspond with the nucleotide numbering for HCV strain HCV-H.

The invention further envisages the above described method for amplification of an HCV NS5B nucleic acid of any one of HCV genotypes 1 to 6 wherein said set of primers is further characterized in that it comprises a sense primer that is capable of annealing to the NS5B genomic region spanning nucleotides 7500 to 7555, wherein the nucleotide positions correspond with the nucleotide numbering for HCV strain HCV-H.

The invention also envisages the above described method for amplification of an HCV NS5B nucleic acid from HCV genotype 1 wherein said set of primers is further characterized in that it comprises a sense primer that is capable of annealing to the NS5B genomic region spanning nucleotides 7536 to 7555, wherein the nucleotide positions correspond with the nucleotide numbering for HCV strain HCV-H.

The invention also envisages the above described method for amplification of an HCV NS5B nucleic acid of any one of HCV genotypes 2 to 6 wherein said set of primers is further characterized in that it comprises a sense primer that is capable of annealing to the NS5B genomic region spanning nucleotides 7500 to 7521, wherein the nucleotide positions correspond with the nucleotide numbering for HCV strain HCV-H.

The invention also envisages the above described method for amplification of an HCV NS5B nucleic acid of any one of HCV genotypes 1 to 6 wherein said set of primers is further characterized in that it comprises a sense primer that is capable of annealing to the NS5B genomic region spanning nucleotides 8258 to 8278, wherein the nucleotide positions correspond with the nucleotide numbering for HCV strain HCV-H.

In a further aspect, the invention relates to a method for amplification of an HCV NS5B nucleic acid of HCV genotype 1 comprising the step of designing a set of primers in the NS5B genomic region, wherein said set of primers for amplifying said HCV NS5B nucleic acid is characterized in that at least one of the primers is an antisense primer that is capable of annealing to the NS5B genomic region spanning nucleotides 8529 to 8546, wherein the nucleotide positions correspond with the nucleotide numbering for HCV strain HCV-H.

The invention also envisages the above method for amplification of an HCV NS5B nucleic acid of HCV genotype 1 wherein said set of primers is further characterized in that it comprises a sense primer that is capable of annealing to the NS5B genomic region spanning nucleotides 7536 to 7555, wherein the nucleotide positions correspond with the nucleotide numbering for HCV strain HCV-H.

In still a further aspect, the invention relates to a method for amplification of an HCV NS5B nucleic acid of HCV genotype 2 comprising the step of designing a set of primers in the NS5B genomic region, wherein said set of primers for amplifying said HCV NS5B nucleic acid is characterized in that at least one of the primers is an antisense primer that is capable of annealing to the NS5B genomic region spanning nucleotides 8620 to 8638, wherein the nucleotide positions correspond with the nucleotide numbering for HCV strain HCV-H.

The invention also envisages the above method for amplification of an HCV NS5B nucleic acid of HCV genotype 2 wherein said set of primers is further characterized in that it comprises a sense primer that is capable of annealing to the NS5B genomic region spanning nucleotides 7500 to 7521, wherein the nucleotide positions correspond with the nucleotide numbering for HCV strain HCV-H.

In still a further aspect, the invention relates to a method for amplification of an HCV NS5B nucleic acid of any one of HCV genotypes 3 to 5 comprising the step of designing a set of primers per genotype in the NS5B genomic region, wherein said set of primers for amplifying said HCV NS5B nucleic acid is characterized in that at least one of the primers is an antisense primer that is capable of annealing to the NS5B genomic region spanning nucleotides 8470 to 8491, wherein the nucleotide positions correspond with the nucleotide numbering for HCV strain HCV-H.

The invention also envisages the abovem method for amplification of an HCV NS5B nucleic acid of any one of HCV genotypes 3 to 5, wherein said set of primers is further characterized in that it comprises a sense primer that is capable of annealing to the NS5B genomic region spanning nucleotides 7500 to 7521, wherein the nucleotide positions correspond with the nucleotide numbering for HCV strain HCV-H.

In still a further aspect, the invention relates to a method for amplification of an HCV NS5B nucleic acid of HCV genotype 6 comprising the step of designing a set of primers in the NS5B genomic region, wherein said set of primers for amplifying said HCV NS5B nucleic acid is characterized in that at least one of the primers is an antisense primer that is capable of annealing to the NS5B genomic region spanning nucleotides 8529 to 8554, wherein the nucleotide positions correspond with the nucleotide numbering for HCV strain HCV-H.

The invention also envisages the above method for amplification of an HCV NS5B nucleic acid of HCV genotype 6, wherein said set of primers is further characterized in that it comprises a sense primer that is capable of annealing to the NS5B genomic region spanning nucleotides 7500 to 7521, wherein the nucleotide positions correspond with the nucleotide numbering for HCV strain HCV-H.

In still a further aspect, a method for amplification of an HCV NS5B nucleic acid of any one of HCV genotypes 1 to 6 comprising the step of designing a set of primers per genotype in the NS5B genomic region, wherein said set of primers for amplifying said HCV NS5B nucleic acid is characterized in that one of the primers is an antisense primer chosen from the group consisting of
- an antisense primer capable of annealing to the NS5B genomic region spanning nucleotides 9276 to 9298,
- an antisense primer capable of annealing to the NS5B genomic region spanning nucleotides 8529 to 8546,
- an antisense primer capable of annealing to the NS5B genomic region spanning nucleotides 8620 to 8638,
- an antisense primer capable of annealing to the NS5B genomic region spanning nucleotides 8470 to 8491,
- an antisense primer capable of annealing to the NS5B genomic region spanning nucleotides 8529 to 8554,
and that the other primer is a sense primer chosen from the group consisting of
- a sense primer capable of annealing to the NS5B genomic region spanning nucleotides 7500 to 7555,
- a sense primer capable of annealing to the NS5B genomic region spanning nucleotides 7536 to 7555,
- a sense primer capable of annealing to the NS5B genomic region spanning nucleotides 7500 to 7521,
- a sense primer capable of annealing to the NS5B genomic region spanning nucleotides 8258 to 8278,
and wherein the nucleotide positions correspond with the nucleotide numbering for HCV strain HCV-H.

A further step in any of the above methods is the amplification of said NS5B nucleic acid with the set of primers as designed in any of these methods.

In any of these methods, said amplification can also be part of a multiplex amplification method.

In another aspect, the invention relates to a method for determining the sequence of an HCV NS5B nucleic acid of any one of HCV genotypes 1 to 6 wherein said NS5B nucleic acid is amplified according to any of the above described methods prior to or concurrent with determining said sequence.

Further, the invention relates to a method for evaluating the sequence variations in an HCV NS5B nucleic acid of any of HCV genotypes 1 to 6 wherein the sequence of said NS5B nucleic acid is determined according to the above described method prior to evaluating the sequence variations. Said sequence variations can be mutations that are induced by HCV antiviral agents. Said sequence variations can be mutations causing HCV drug resistance. Said sequence variations can also be genotype-specific nucleotide variations.

In any of these methods, said amplification can be a one-step RT-PCR and said HCV NS5B nucleic acid can be present in a biological sample.

Further, the invention also relates to a recombinant vector comprising an NS5B fragment amplified according to any of the above described methods.

In another aspect, the invention relates to a set of primers for amplification of an HCV NS5B nucleic acid of any one of HCV genotypes 1 to 6, said set of primers characterized in that at least one of the primers is an antisense primer that is capable of annealing to the HCV NS5B genomic region spanning nucleotides 9276 to 9298, wherein the nucleotide positions correspond with the nucleotide numbering for HCV strain HCV-H.

The invention further relates to the above set of primers for amplification of an HCV NS5B nucleic acid of any one of HCV genotypes 1 to 6, said set of primers further characterized in that it comprises a sense primer that is capable of annealing to the HCV NS5B genomic region spanning nucleotides 7500 to 7555, wherein the nucleotide positions correspond with the nucleotide numbering for HCV strain HCV-H.

The invention also relates to the above set of primers for amplification of an HCV NS5B nucleic acid from HCV genotype 1, said set of primers further characterized in that it comprises a sense primer that is capable of annealing to the HCV NS5B genomic region spanning nucleotides 7536 to 7555, wherein the nucleotide positions correspond with the nucleotide numbering for HCV strain HCV-H.

The invention also relates to the above set of primers for amplification of an HCV NS5B nucleic acid of any one of HCV genotypes 2 to 6, said set of primers further characterized in that it comprises a sense primer that is capable of annealing to the HCV NS5B genomic region spanning nucleotides 7500 to 7521, wherein the nucleotide positions correspond with the nucleotide numbering for HCV strain HCV-H.

The invention also relates to the above set of primers for amplification of an HCV NS5B nucleic acid of any one of HCV genotypes 1 to 6, said set of primers further characterized in that it comprises a sense primer that is capable of annealing to the HCV NS5B genomic region spanning nucleotides 8258 to 8278, wherein the nucleotide positions correspond with the nucleotide numbering for HCV strain HCV-H.

In a further aspect, the invention relates to a set of primers for amplification of an HCV NS5B nucleic acid of HCV genotype 1, said set of primers characterized in that at least one of the primers is an antisense primer that is capable of annealing to the HCV NS5B genomic region spanning nucleotides 8529 to 8546, wherein the nucleotide positions correspond with the nucleotide numbering for HCV strain HCV-H.

The invention further relates to the above set of primers for amplification of an HCV NS5B nucleic acid of HCV genotype 1, said set of primers further characterized in that it comprises a sense primer that is capable of annealing to the HCV NS5B genomic region spanning nucleotides 7536 to 7555, wherein the nucleotide positions correspond with the nucleotide numbering for HCV strain HCV-H.

In still a further aspect, the invention relates to a set of primers for amplification of an HCV NS5B nucleic acid of HCV genotype 2, said set of primers characterized in that at least one of the primers is an antisense primer that is capable of annealing to the HCV NS5B genomic region spanning nucleotides 8620 to 8638, wherein the nucleotide positions correspond with the nucleotide numbering for HCV strain HCV-H.

The invention also relates to the above set of primers for amplification of an HCV NS5B nucleic acid of HCV genotype 2, said set of primers further characterized in that it comprises a sense primer that is capable of annealing to the HCV NS5B genomic region spanning nucleotides 7500 to 7521, wherein the nucleotide positions correspond with the nucleotide numbering for HCV strain HCV-H.

In still a further aspect, the invention relates to a set of primers for amplification of an HCV NS5B nucleic acid of any one of HCV genotypes 3 to 5, said set of primers characterized in that at least one of the primers is an antisense primer that is capable of annealing to the HCV NS5B genomic region spanning nucleotides 8470 to 8491, wherein the nucleotide positions correspond with the nucleotide numbering for HCV strain HCV-H.

The invention also relates to the above set of primers for amplification of an HCV NS5B nucleic acid of any one of HCV genotypes 3 to 5, said set of primers further characterized in that it comprises a sense primer that is capable of annealing to the HCV NS5B genomic region spanning nucleotides 7500 to 7521, wherein the nucleotide positions correspond with the nucleotide numbering for HCV strain HCV-H.

In still a further aspect, the invention relates to a set of primers for amplification of an HCV NS5B nucleic acid of HCV genotype 6, said set of primers characterized in that at least one of the primers is an antisense primer that is capable of annealing to the HCV NS5B genomic region spanning nucleotides 8529 to 8554, wherein the nucleotide positions correspond with the nucleotide numbering for HCV strain HCV-H.

The invention also relates to the above set of primers for amplification of an HCV NS5B nucleic acid of HCV genotype 6, said set of primers further characterized in that it comprises a sense primer that is capable of annealing to the HCV NS5B genomic region spanning nucleotides 7500 to 7521, wherein the nucleotide positions correspond with the nucleotide numbering for HCV strain HCV-H.

In still a further aspect, the invention relates to a set of primers for amplification of an HCV NS5B nucleic acid of any one of HCV genotypes 1 to 6, said set of primers characterized in that one of the primers is an antisense primer chosen from the group consisting of
- an antisense primer capable of annealing to the NS5B genomic region spanning nucleotides 9276 to 9298,
- an antisense primer capable of annealing to the NS5B genomic region spanning nucleotides 8529 to 8546,
- an antisense primer capable of annealing to the NS5B genomic region spanning nucleotides 8620 to 8638,
- an antisense primer capable of annealing to the NS5B genomic region spanning nucleotides 8470 to 8491,
- an antisense primer capable of annealing to the NS5B genomic region spanning nucleotides 8529 to 8554,
   and that the other primer is a sense primer chosen from the group consisting of
- a sense primer capable of annealing to the NS5B genomic region spanning nucleotides 7500 to 7555,
- a sense primer capable of annealing to the NS5B genomic region spanning nucleotides 7536 to 7555,
- a sense primer capable of annealing to the NS5B genomic region spanning nucleotides 7500 to 7521,
- a sense primer capable of annealing to the NS5B genomic region spanning nucleotides 8258 to 8278,
and wherein the nucleotide positions correspond with the nucleotide numbering for HCV strain HCV-H.

In another aspect, the invention relates to the use of any of the above sets of primers for the manufacture of a kit for amplification of an HCV NS5B nucleic acid of any one of HCV genotypes 1 to 6.

In still another aspect, the invention relates to a kit for amplification of an HCV NS5B nucleic acid of any one of HCV genotypes 1 to 6, said kit comprising any of the above sets of primers. The above described kit can be used for multiplex amplification. The invention also relates to a kit for assaying sequence variations in an HCV NS5B nucleic acid of any one of HCV genotypes 1 to 6, said kit comprising any of the above set of primers, wherein said sequence variations can be mutations that are induced by HCV antiviral agents, mutations causing HCV drug resistance, or genotype-specific nucleotide variations.

### DETAILED DESCRIPTION OF THE INVENTION

A robust and consistent amplification and sequencing methodology for analyzing sequence variations in potential HCV anti-viral genomic targets is a primary tool for drug resistance profiling. Such a methodology is useful for monitoring patients under treatment with novel antiviral drugs and therapeutic regimens, such as those targeting the HCV NS3 protease and the HCV NS5B RNA polymerase. The current invention provides the basis for such methodologies and tools relating to the HCV NS5B polymerase.

A plethora of primer sequences for amplifying and sequencing portions of the HCV genome have been disclosed not only in public databases but also in, e.g., International Patent Publications WO94/12670, WO94/25601, WO96/13590, WO03/020970, WO03/077729. However, these known primers do not allow for amplifying, under one set of conditions, the full length of NS5B of all HCV genotypes, which is needed to perform a complete nucleic acid analysis.

Assessment of drug resistance development and defining the effects of inhibitors on different viral genotypes are now viewed as integral parts of antiviral drug discovery, even in the early stages of drug development. The early detection of such variants and evaluation of the drug-resistance profile of these antiviral agents is considered crucial for identifying inhibitors with a higher probability of clinical success. The current invention provides diagnostic tools and methodologies for analyzing sequence variations in potential HCV anti-viral genomic targets. More specifically, the present invention provides for a robust and consistent amplification and sequencing methodology for analyzing drug resistance mutants in the NS5B RNA polymerase region of genotypes 1 to 6 of the hepatitis C virus. This methodology is useful for the sequential analysis and follow-up of patients under treatment with novel antiviral drugs targeting HCV NS5B.

In particular, uniform PCR protocols for the simultaneous amplification of the full length NS5B regions of the 6 major HCV genotypes were developed. The presently developed methodology takes into account the extensive genotype and subtype variability of the HCV genome. In order to cope with the variability of the different HCV genotypes, different sets of forward and reverse primers were designed per genotype to amplify the complete NS5B region. It should be clear to the skilled man that such genotype-specific primer design was not merely based on a general alignment of all known HCV genotype sequences and subsequent identification of homologous regions. Notably, the existing sequence polymorphism amongst the different subtypes of a certain genotype is very high and rendered primer design covering all subtypes within a certain genotype very difficult. The primer sets were further optimized to allow their use for amplification of each region and genotype under one set of conditions, i.e. under one specific amplification protocol. This was done for "ease of use" purposes, especially in view of time managment and reduction of reagent costs associated with the procedure. For further "ease of use", a single extraction method and one-step PCT protocol was optimized for all genotypes. The final protocol resulted in the amplification of one full length NS5B fragment or, in the alternative, two overlapping NS5B fragments covering the complete NS5B gene.

In conclusion, based on the above findings and as further demonstrated by the examples, it should be clear that the simultaneous evaluation of sequence variations within the complete NS5B polymerase gene for all HCV genotypes by using the presently claimed amplification and sequencing methodology is truly advantageous as compared to using multiple sets of existing primers in different conditions and amplification protocols, resulting in a multitude of small amplified fragments scattered over (part of) the HCV NS5B genomic region.

In a first aspect, the present invention relates to a method for amplification of an HCV NS5B nucleic acid of any one of HCV genotypes 1 to 6 comprising the step of designing a set of primers per genotype in the NS5B genomic region, wherein said set of primers for amplifying said HCV NS5B nucleic acid is characterized in that at least one of the primers is an antisense primer that is capable of annealing to the NS5B genomic region spanning nucleotides 9276 to 9298, wherein the nucleotide positions correspond with the nucleotide numbering for HCV strain HCV-H.

The invention further envisages the above described methods for amplification of an HCV NS5B nucleic acid of any one of HCV genotypes 1 to 6 wherein said set of primers is further characterized in that it comprises a sense primer that is capable of annealing to the NS5B genomic region spanning nucleotides 7500 to 7555, wherein the nucleotide positions correspond with the nucleotide numbering for HCV strain HCV-H.

The invention also envisages the above described methods for amplification of an HCV NS5B nucleic acid from HCV genotype 1 wherein said set of primers is further characterized in that it comprises a sense primer that is capable of annealing to the NS5B genomic region spanning nucleotides 7536 to 7555, wherein the nucleotide positions correspond with the nucleotide numbering for HCV strain HCV-H.

The invention also envisages the above described methods for amplification of an HCV NS5B nucleic acid of any one of HCV genotypes 2 to 6 wherein said set of primers is further characterized in that it comprises a sense primer that is capable of annealing to the NS5B genomic region spanning nucleotides 7500 to 7521, wherein the nucleotide positions correspond with the nucleotide numbering for HCV strain HCV-H.

The invention also envisages the above described methods for amplification of an HCV NS5B nucleic acid of any one of HCV genotypes 1 to 6 wherein said set of primers is further characterized in that it comprises a sense primer that is capable of annealing to the NS5B genomic region spanning nucleotides 8258 to 8278, wherein the nucleotide positions correspond with the nucleotide numbering for HCV strain HCV-H.

In a further aspect, the invention relates to a method for amplification of an HCV NS5B nucleic acid of HCV genotype 1 comprising the step of designing a set of primers in the NS5B genomic region, wherein said set of primers for amplifying said HCV NS5B nucleic acid is characterized in that at least one of the primers is an antisense primer that is capable of annealing to the NS5B genomic region spanning nucleotides 8529 to 8546, wherein the nucleotide positions correspond with the nucleotide numbering for HCV strain HCV-H.

The invention also envisages the above method for amplification of an HCV NS5B nucleic acid of HCV genotype 1 wherein said set of primers is further characterized in that it comprises a sense primer that is capable of annealing to the NS5B genomic region spanning nucleotides 7536 to 7555, wherein the nucleotide positions correspond with the nucleotide numbering for HCV strain HCV-H.

In still a further aspect, the invention relates to a method for amplification of an HCV NS5B nucleic acid of HCV genotype 2 comprising the step of designing a set of primers in the NS5B genomic region, wherein said set of primers for amplifying said HCV NS5B nucleic acid is characterized in that at least one of the primers is an antisense primer that is capable of annealing to the NS5B genomic region spanning nucleotides 8620 to 8638, wherein the nucleotide positions correspond with the nucleotide numbering for HCV strain HCV-H.

The invention also envisages the above method for amplification of an HCV NS5B nucleic acid of HCV genotype 2 wherein said set of primers is further characterized in that it comprises a sense primer that is capable of annealing to the NS5B genomic region spanning nucleotides 7500 to 7521, wherein the nucleotide positions correspond with the nucleotide numbering for HCV strain HCV-H.

In still a further aspect, the invention relates to a method for amplification of an HCV NS5B nucleic acid of any one of HCV genotypes 3 to 5 comprising the step of designing a set of primers per genotype in the NS5B genomic region, wherein said set of primers for amplifying said HCV NS5B nucleic acid is characterized in that at least one of the primers is an antisense primer that is capable of annealing to the NS5B genomic region spanning nucleotides 8470 to 8491, wherein the nucleotide positions correspond with the nucleotide numbering for HCV strain HCV-H.

The invention also envisages the abovem method for amplification of an HCV NS5B nucleic acid of any one of HCV genotypes 3 to 5, wherein said set of primers is further characterized in that it comprises a sense primer that is capable of annealing to the NS5B genomic region spanning nucleotides 7500 to 7521, wherein the nucleotide positions correspond with the nucleotide numbering for HCV strain HCV-H.

In still a further aspect, the invention relates to a method for amplification of an HCV NS5B nucleic acid of HCV genotype 6 comprising the step of designing a set of primers in the NS5B genomic region, wherein said set of primers for amplifying said HCV NS5B nucleic acid is characterized in that at least one of the primers is an antisense primer that is capable of annealing to the NS5B genomic region spanning nucleotides 8529 to 8554, wherein the nucleotide positions correspond with the nucleotide numbering for HCV strain HCV-H.

The invention also envisages the above method for amplification of an HCV NS5B nucleic acid of HCV genotype 6, wherein said set of primers is further characterized in that it comprises a sense primer that is capable of annealing to the NS5B genomic region spanning nucleotides 7500 to 7521, wherein the nucleotide positions correspond with the nucleotide numbering for HCV strain HCV-H.

In still a further aspect, a method for amplification of an HCV NS5B nucleic acid of any one of HCV genotypes 1 to 6 comprising the step of designing a set of primers per genotype in the NS5B genomic region, wherein said set of primers for amplifying said HCV NS5B nucleic acid is characterized in that one of the primers is an antisense primer chosen from the group consisting of
- an antisense primer capable of annealing to the NS5B genomic region spanning nucleotides 9276 to 9298,
- an antisense primer capable of annealing to the NS5B genomic region spanning nucleotides 8529 to 8546,
- an antisense primer capable of annealing to the NS5B genomic region spanning nucleotides 8620 to 8638,
- an antisense primer capable of annealing to the NS5B genomic region spanning nucleotides 8470 to 8491,
- an antisense primer capable of annealing to the NS5B genomic region spanning nucleotides 8529 to 8554,
   and that the other primer is a sense primer chosen from the group consisting of
- a sense primer capable of annealing to the NS5B genomic region spanning nucleotides 7500 to 7555,
- a sense primer capable of annealing to the NS5B genomic region spanning nucleotides 7536 to 7555,
- a sense primer capable of annealing to the NS5B genomic region spanning nucleotides 7500 to 7521,
- a sense primer capable of annealing to the NS5B genomic region spanning nucleotides 8258 to 8278,
and wherein the nucleotide positions correspond with the nucleotide numbering for HCV strain HCV-H.

To further clarify the scope of the current invention, the method for amplification of an HCV NS5B nucleic acid of any one of HCV genotypes 1 to 6 can comprise the steps of
(i) designing a set of primers per genotype in the NS5B genomic region as described in any of the above methods, and
(ii) optionally selecting a set of primers of (i) that are effectively annealing to and capable of amplifying said NS5B nucleic acid; and
(iii) amplification of said NS5B nucleic acid with the set of primers designed in (i) or selected in (ii);
wherein the nucleotide positions correspond with the nucleotide numbering for HCV strain HCV-H.

Consistency in numbering of nucleotides in the current invention is based on taking HCV strain HCV-H as a reference strain (Inchauspe et al. 1991). The complete genome sequence of HCV-H, which is a type 1a isolate, is available at GenBank (Accession Number M67463). The HCV-H sequence consists of 9416 nucleotides including the 5' and 3' untranslated or non-coding regions (UTR; NCR). Nucleotide numbering starts at the first nucleotide with position 1. The adenine residue at position 342 is the first residue of the ATG start codon that initiates the long HCV polyprotein of 3011 amino acids. As type 1a isolates contain 1 extra amino acid in the NS5A region as compared to type 1b isolates, coding sequences of type 1a and 1b have identical numbering in the Core, E1, E2, NS2, NS3 and NS4 region, but a slightly different numbering in the NS5 region. Type 2 isolates have 4 extra amino acids in the E2 region, and 17 or 18 extra amino acids in the NS5 region compared to type 1 isolates, and will differ in numbering from type 1 isolates in the NS2, NS3, NS4 and NS5 regions. Similar insertions compared with type 1 (but of a different size) can also be observed in genotype 3 to 6 sequences which affect the numbering of the respective types accordingly. Other insertions or deletions may be readily observed in genotype 1 to 6 sequences after alignment with the reference strain HCV-H. A given genomic region of an HCV genotype or isolate of interest can always and easily be placed at the same relative position of the corresponding region of HCV-H. For example, an insertion or deletion of one or more nucleotides in the Core region of a given HCV isolate changes the nucleotide numbering in the NS5 region relative to HCV-H. Nevertheless, the NS5 region of the HCV isolate can be integrally aligned with the NS5 region of HCV-H. Consistency in nucleotide numbering means that the nucleotide numbering of the NS5 region of HCV-H is taken as the standard numbering when refering to nucleotides of the NS5 region of another HCV isolate.

"HCV genotypes 1 to 6" refer to all HCV variants characterized since the initial molecular characterization of HCV in 1989. "HCV genotype 1", "HCV genotype 2", "HCV genotypes 3 to 5" and "HCV genotype 6" refer to subgroups of said HCV variants. HCV variants are classified into 6 HCV major genotypes and a series of subtypes (up to 80) by phylogenetic analysis of Core/E1 and NS5B subgenomic sequences as well as of complete genome sequences. A comprehensive listing of all currently classified HCV variants and provisionally assigned HCV genotypes/subtypes is provided in Table 1 and 2 of Simmonds et al. (2005). HCV variants may also include HCV "quasispecies", which are a group of related but distinct HCV variants within an infected individual. This heterogeneity is an inherent characteristic of RNA viruses.

With "HCV NS5B nucleic acid or NS5B genomic region" is meant any HCV NS5B genomic sequence. Said HCV NS5B genomic sequence can be part of a larger HCV genomic sequence.

Amplification of said HCV NS5B genomic sequence according to the invention results in an amplified HCV NS5B nucleic acid fragment starting at around nucleotide position 7500 and ending at around nucleotide position 9298, or fragments thereof, wherein the nucleotide positions correspond with the nucleotide numbering for HCV strain HCV-H as outlined above. In particular, HCV NS5B nucleic acid fragments starting at nucleotides 7500 to 7555 and ending at nucleotides 9276 to 9298 are meant, for example starting at nucleotides 7500 to 7517, 7500 to 7518, 7500 to 7521, 7536 to 7555, and ending at nucleotides 9276 to 9296, 9276 to 9297, 9276 to 9298. HCV NS5B nucleic acid fragments starting at around nucleotide position 8258 to around nucleotide position 9298 are also included. In particular, HCV NS5B nucleic acid fragments starting at nucleotides 8258 to 8278 and ending at nucleotides 9276 to 9298 are meant, for example starting at nucleotides 8259 to 8278 and ending at nucleotides 9276 to 9296, 9276 to 9297, 9276 to 9298.

Amplification of said HCV NS5B genomic sequence according to the present invention also results in an amplified HCV NS5B nucleic acid fragment starting at around nucleotide position 7500 and ending at around nucleotide position 8546, or fragments thereof, wherein the nucleotide positions correspond with the nucleotide numbering for HCV strain HCV-H as outlined above. In particular, HCV NS5B nucleic acid fragments starting at nucleotides 7536 to 7555 and ending at nucleotides 8529 to 8546 are meant.

Amplification of said HCV NS5B genomic sequence according to the present invention further results in an amplified HCV NS5B nucleic acid fragment starting at around nucleotide position 7500 and ending at around nucleotide position 8638, or fragments thereof, wherein the nucleotide positions correspond with the nucleotide numbering for HCV strain HCV-H as outlined above. In particular, HCV NS5B nucleic acid fragments starting at nucleotides 7500 to 7521 and ending at nucleotides 8620 to 8638 are meant.

Amplification of said HCV NS5B genomic sequence according to the present invention further results in an amplified HCV NS5B nucleic acid fragment starting at around nucleotide position 7500 and ending at around nucleotide position 8491, or fragments thereof, wherein the nucleotide positions correspond with the nucleotide numbering for HCV strain HCV-H as outlined above. In particular, HCV NS5B nucleic acid fragments starting at nucleotides 7500 to 7521 and ending at nucleotides 8470 to 8491 are meant, for example starting at nucleotides 7500 to 7517, 7500 to 7518, 7500 to 7521 and ending at nucleotides 8470 to 8488, 8470 to 8491, 8473 to 8491.

Amplification of said HCV NS5B genomic sequence according to the present invention further results in an amplified HCV NS5B nucleic acid fragment starting at around nucleotide position 7500 and ending at around nucleotide position 8554, or fragments thereof, wherein the nucleotide positions correspond with the nucleotide numbering for HCV strain HCV-H as outlined above. In particular, HCV NS5B nucleic acid fragments starting at nucleotides 7500 to 7521 and ending at nucleotides 8529 to 8554 are meant, for example starting at nucleotides 7500 to 7517, 7500 to 7521 and ending at nucleotides 8529 to 8554, 8534 to 8554.

It should be clear that the length of the resulting amplified HCV NS5B nucleic acid fragments is not restricted to the above described examples. In fact, it should be clear by the skilled man that the exact start and endpoints of the HCV NS5B nucleic acid fragments are determined by the primers used for amplifying said fragments (see *infra*).

A "primer" is the at least one oligonucleotide in a nucleic acid amplification reaction mixture that is required to obtain specific amplification of a target nucleic acid. Nucleic acid amplification can be linear or exponential and can result in an amplified single stranded nucleic acid derived from a single- or double-stranded nucleic acid or can result in both strands of a double-stranded nucleic acid. Specificity of a primer in directing amplification of a nucleic acid can be improved by introducing modified nucleotides in said primer or by adapting hybridization or annealing conditions (see e.g. Example 1). The fact that a primer does not have to match exactly with the corresponding template or target sequence to warrant specific amplification of said template or target sequence is amply documented in literature (for instance: Kwok et al. 1990). In this context, the expression "capable of annealing to" refers to allowing the amplification of the target nucleic acid for which specific primers are designed. The term "oligonucleotide" referred to herein is meant to include all nucleic acid molecules comprising or consisting of a part of the HCV nucleic acids according to the invention. The lower size limit of an oligonucleotide is a nucleic acid comprising or consisting of at least 8 contiguous nucleotides of HCV nucleic acids. An oligonucleotide thus can comprise or consist of at least and/or comprise or consist of up to 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70 or more contiguous nucleotides of HCV nucleic acids. Primers as short as 8 nucleotides in length have been applied successfully in directing specific amplification of a target nucleic acid molecule (for instance: Majzoub et al. 1983).

A "sense primer" or a forward primer is a primer that will direct amplification by annealing to the antisense strand of a target nucleic acid, and will generate the sense strand of an amplification product.

An "antisense primer" or a reverse primer is a primer that will direct amplification by annealing to the sense strand of a target nucleic acid, and will generate the antisense strand of an amplification product.

A target nucleic acid refers to the nucleic acid that is to be amplified. A target region refers to a region of the nucleic acid that is to be amplified.

A polynucleic acid or a nucleic acid or an oligonucleotide of the present invention may be of genomic origin, RNA, DNA, cDNA or a synthetic polynucleic acid and may comprise modified nucleotides, wherein said modification can occur in the nucleotide base or the nucleotide sugar. Any of the modifications can be introduced in a nucleic acid or oligonucleotide to increase/decrease stability and/or reactivity of the nucleic acid or oligonucleotide and/or for other purposes such as labeling of the nucleic acid or oligonucleotide. Modified nucleotides include phophorothioates, alkylphophorothioates, methylphosphonate, phosphoramidate, peptide nucleic acid monomers and locked nucleic acid monomers. A polynucleic acid or a nucleic acid can be single- or double-stranded or may be a triplex-forming nucleic acid. The terms nucleic acid and polynucleic acid are used interchangeably herein.

"Amplification" is meant to include all methods resulting in multiplication of the number of a target nucleic acid. Nucleotide sequence amplification methods may include but are not limited to the polymerase chain reaction (PCR; DNA amplification), strand displacement amplification (SDA; DNA amplification), ligase chain reaction (LCR; DNA amplification), transcription-based amplification system (TAS; RNA amplification), self-sustained sequence replication (3SR; RNA amplification), nucleic acid sequence-based amplification (NASBA; RNA amplification), transcription-mediated amplification (TMA; RNA amplification), Qbeta-replicase-mediated amplification and run-off transcription. DNA sequencing (classical, such as based on Sanger reaction, or via PCR) is herewith also included as an amplification method (see further). During amplification, the amplified products can be conveniently labeled either using labeled primers or by incorporating labeled nucleotides. Labels may be isotopic (³²P, ³⁵S, etc.) or non-isotopic (biotin, digoxigenin, etc.).

The most widely spread nucleotide sequence amplification technique is PCR. Basically, two primers, a sense and an antisense primer are annealed to a denatured DNA substrate and extended by a thermostable DNA polymerase. The latter allows rapid and repeated thermal cycling (denaturing/annealing/extension in three-step PCR; denaturing/annealing + extension in two-step PCR). The target DNA is exponentially amplified. The amplification reaction is repeated between 20 and 70 times, advantageously between 25 and 45 times. Many methods rely on PCR including AFLP (amplified fragment length polymorphism), IRS-PCR (interspersed repetitive sequence PCR), iPCR (inverse PCR), RAPD (rapid amplification of polymorphic DNA), RT-PCR (reverse transcription PCR) and real-time PCR. RT-PCR can be performed with a single thermostable enzyme having both reverse transcriptase and DNA polymerase activity (Myers and Gelfand 1991). Alternatively, two enzymes (reverse transcriptase and thermostable DNA polymerase) are possible (Cusi et al. 1994). With "single-tube RT-PCR" or "one-step RT-PCR" is meant RT-PCR in which all components of the RT and PCR are mixed in one tube prior to starting the reactions and in which RT and PCR are thus carried out sequentially in one tube. With "two-step RT-PCR" is meant RT-PCR in which the components of the RT and PCR are respectively separated in two tubes prior to starting the reaction and in which RT and PCR are thus carried out sequentially in two tubes.

Multiplex PCR, another variant of PCR which enables simultaneous amplification of many targets of interest in one reaction by using more than one pair of primers, is herewith also included as an amplification method. Examples of said multiplex PCR include but are not limited to simultaneous amplification in one reaction of a single HCV NS5B nucleic acid fragment for multiple HCV genotypes, or of multiple NS5B fragments for a single HCV genotype, or of multiple NS5B fragments for multiple HCV genotypes according to the current invention. In particular, said multiplex PCR may further include primers for simultaneous amplification of any other HCV or non-HCV nucleic acid target sequence, such as a HCV NS3 nucleic acid sequence, a HBV nucleic acid sequence, a HIV nucleic acid sequence etc.

The invention further relates to any of the methods as described above wherein said amplification can be part of a multiplex amplification method.

In another aspect, the invention relates to a method for determining the sequence of an HCV NS5B nucleic acid of any one of HCV genotypes 1 to 6 wherein said NS5B nucleic acid is amplified according to any of the above described methods prior to or concurrent with determining said sequence.

Further, the invention relates to a method for evaluating the sequence variations in an HCV NS5B nucleic acid of any of HCV genotypes 1 to 6 wherein the sequence of said NS5B nucleic acid is determined according to the above described method prior to evaluating the sequence variations. More specifically, said sequence variations can be mutations that are induced by HCV antiviral agents. More specifically, said sequence variations can be mutations causing HCV drug resistance. Said sequence variations can also be genotype-specific nucleotide variations. The evaluation of sequence variations, more specifically the discrimination between either HCV drug resistance mutations or genotype-specific nucleotide variations, can occur simultaneously.

With the term "sequence variations" or "nucleotide variations" is meant at least one nucleotide of an HCV nucleic acid or genomic region according to the invention that is different from the corresponding nucleotide in another HCV nucleic acid sequence to which the comparison is made. "Corresponding nucleotide" in this context is referring to a nucleotide at the same relative position in the HCV genome (whether noted as RNA or DNA) wherein the relative position takes into account the possible occuring genotype-, subtype-, or isolate-specific insertions and/or deletions (see for instance section on Genotype-specific insertions and deletions on page 198 of Maertens and Stuyver 1997); a corresponding nucleotide can also be present in a nucleic acid that is the complement of the nucleic acid to which the comparison is made. Differences between nucleotide sequences can easily be determined by the skilled person, for instance after aligning said sequences.

With the term "genotype-specific nucleotide variations" is meant at least one nucleotide of an HCV nucleic acid or genomic region according to the invention that is unique to or specific to any genotype, subtype or isolate of an HCV classifying in any of the known genotype classes 1 to 6. "Genotype-specific" is referring to the specificity or uniqueness of a nucleotide to an HCV nucleotide sequence belonging to any of HCV genotypes 1 to 6. The term "genotype-specific" is generally accepted as can for instance be derived from EP 0 637 342 B1 or any of US 6,548,244, US 5,882,852 or US 5,514,539. Said unique or specific nucleotides can occur as a single nucleotide or a set of nucleotides specific to a nucleic acid sequence of any of the HCV types identified to date. The specific or unique nucleotides of said set can form a contiguous sequence or can be dispersed in a larger region of a nucleic acid sequence of any of the HCV types identified to date.

With "HCV antiviral agents" are meant any nucleoside or nucleotide analogs as well as immunological reagents and chemical, proteinaceous and nucleic acid agents which inhibit or otherwise interfere with HCV viral replication, maintenance, infection, assembly or release. HCV antiviral agents or inhibitors may include but are not limited to HCV NS5B polymerase inhibitors that currently can be broadly divided into three categories: (i) nucleoside analogues, (ii) pyrophosphate mimics, and (iii) non-nucleoside inhibitors (reviewed in: Beaulieu and Tsantrizos 2004). Most nucleoside analogues, such as those having a 2'-methylribose structure, act as chain terminators of the polymerase reaction (Carroll et al. 2003). The pyrophosphate mimics, exemplified by a series of diketo acids and hydroxypyrimidine carboxylic acids that selectively bind the NS5B active site divalent cations, act as product-like inhibitors of the polymerase reaction (Summa et al. 2004). The non-nucleoside inhibitors are by far the most diverse class of inhibitors and represent compounds that bind to distinct pockets on the HCV polymerase: the thiophene 2-carboxylic acids, phenylalanine derivatives, and cyclopentyl dihydropyran-2-ones classes of compounds all bind to a pocket with a central Met⁴²³ residue (Love et al. 2003; Wang et al. 2003). Another class of compounds is exemplified by benzothiadiazines that give rise to resistant replicon mutants at position Met⁴¹⁴ (Nguyen et al. 2003; Tomei et al. 2004). Benzimidazole 5-carboxamide inhibitors have also been extensively pursued as HCV NS5B-specific inhibitors (Kukolj et al. 2005; International patent applications WO 03/007945 and WO 03/010141), which inhibit productive binding of RNA template/primer substrate. With "HCV antiviral agents" are also meant combinations of compounds binding to different sites of one HCV target or to other HCV targets, such as HCV protease inhibitors.

The phrase "mutations that are induced by HCV antiviral agents" relates to a nucleotide variation in an HCV gene encoding an HCV structural or non-structural protein caused by said HCV antiviral agents as described above. More specifically, the mutation may or may not result in an altered amino acid sequence (amino acid addition, substitution and/or deletion) in the targeted region. Viral variants carrying said mutations may or may not exhibit reduced sensitivity to one or more of said HCV antiviral agents as described above. The term "mutation" is to be read in its broadest context and may include one or more mutations. The phrase "mutations causing HCV drug resistance" relates to acquired mutations induced by said HCV antiviral agents as described above or to mutations that spontaneously occurred, causing HCV drug resistance. In all cases, viral variants carrying HCV drug resistance mutations exhibit reduced sensitivity to one or more of said HCV antiviral agents as described above. For example, HCV drug resistant mutations that emerged with the benzimidazole 5-carboxamide class of NS5B inhibitors were found at three amino acid positions, each conferring different levels of resistance to this drug class: Pro⁴⁹⁵ with substitutions to Ser, Leu, Ala or Thr; Pro⁴⁹⁶ with substitutions to Ser or Ala; and Val⁴⁹⁹ with substitutions to Ala (Tomei et al. 2003; Kukolj et al. 2005). Also, the occurrence of certain HCV drug resistance mutations may be correlated with a certain HCV genotype.

A large number of assays capable of detecting nucleotide sequences and nucleotide sequence variations is currently available. These assays can identify specific mutations, single nucleotide polymorphisms (SNPs), genotype-specific nucleotides or the like. Some of these assays are based on physical methods whereas others use enzymatic approaches.

With "physical detection methods" is meant in the present context methods of nucleotide sequence variation detection that require one or more physical processes for detection although not excluding the enzymatic process of prior amplification of the target DNA sequence comprising one or more nucleotide sequence polymorphisms. Said physical processes include electrophoresis, chromatography, spectrometry, optical signal sensing and spectroscopy. The nucleotide sequence variations to be detected more specifically are the polymorphisms occurring in the nucleic acids of different HCV genotypes, -subtypes, or -isolates.

Physical nucleotide sequence variation detection assays include electrophoretic methods such as SSCP (single stranded conformation polymorphism), CDCE (constant denaturant capillary electrophoresis), CDGE (constant denaturant gel electrophoresis), DGGE (denaturing gradient gel electrophoresis), TGCE (thermal gradient capillary electrophoresis), DGCE (double gradient capillary electrophoresis), nonisocratic CZE (capillary zone electrophoresis), TDGS (two-dimensional gene scanning), CSGE (conformation sensitive gel electrophoresis), MADGE (microplate array diagonal gele electrophoresis), DSCA (double stand conformation analysis), HMA (heteroduplex mobility assay), HTA (heteroduplex tracking assay); chromatographic methods include DHPLC (denaturing high performance liquid chromatography). Physical nucleotide sequence variation detection assays may be effective for identification of known or new mutations and may require confirmation by direct DNA sequencing. Probes or primers utilized in any of these physical nucleotide sequence variation detection assays may be modified to increase their discriminatory power, such modifications including the addition of a GC-clamp (i.e., an artificial high-melting domain) to a probe or primer.

MALDI-TOF MS (matrix-assisted laser desorption-ionization time-of-flight mass spectrometry) has been succesfully used both as a direct DNA sequencing tool for DNA fragments under 100 bp and as a tool for detection of single nucleotide polymorphisms. Hybridization of allele-specific PNA-oligomers (peptide nucleic acid) with single stranded target DNA was proven to be highly compatible with MALDI-TOF MS analysis (Griffin and Smith 2000, and references therein).

With "enzymatic approaches for the generation of products signaling nucleotide sequence variations" is meant in the present context approaches relying on the activity of one or more enzymes for generation of said signaling products. Enzymes include DNA restriction endonucleases, DNA polymerases, DNA ligases, DNA/RNA structure-specific endonucleases, DNA/RNA flap endonucleases, DNA exonucleases and reverse transcriptases (RTs). Enzymatic approaches usually require a physical process (e.g. as described supra) for detection of the enzymatically produced signal. Said enzymatic approaches include RFLP (restriction fragment length polymorphism), AFLP (amplified fragment length polymorphism), ASO-PCR (allele-specific oligonucleotide PCR), real-time PCR, LCR (ligase chain reaction) and any variation thereof, LDR (ligase detection reaction), CFLP (Cleavase fragment length polymorphism), EMD (enyzmatic mutation detection), NIRCA (non-isotropic RNase cleavage assay), Invader™ assay and its modifications, MIDAS (mutation identification DNA analysis system), ddF (dideoxy fingerprinting), Bi-ddF (bidirectional ddF), dnF (denaturing ddF), BESS (base excision sequence scanning) and DNA minisequencing or DNA sequencing. Probes or primers utilized in any of these enzymatically-based nucleotide sequence polymorphism detection assays may carry specific modifications in order to detect the target nucleotide sequence polymorphism. Such modifications include labeling with a single label, with two different labels (for instance two fluorophores or one fluorophore and one quencher), the attachment of a different 'universal' tail to two probes or primers hybridizing adjacent or in close proximity to each other with the target nucleotide sequence, the incorporation of a target-specific sequence in a hairpin probe or primer (for instance Molecular Beacon-type primer), the tailing of such a hairpin probe or primer with a 'universal' tail (for instance Sunrise-type probe and Amplifluor™-type primer). A special type of hairpin probe/primer incorporates in the hairpin a sequence capable of hybridizing to part of the newly amplified target DNA. Amplification of the hairpin is prevented by the incorporation of a blocking nonamplifiable monomer (such as hexethylene glycol). A fluorescent signal is generated after opening of the hairpin due to hybridization of the hairpin loop with the amplified target DNA. This type of hairpin probe/primer is know as scorpion primers (Whitcombe et al. 1999). Another special type of probe is a padlock probe (or circularizable probe or open circle probe or C-probe) that are used in RCA (rolling circle amplification). The technique of CFLP fingerprinting has already been applied to perform HCV genotyping (Sreevatsan et al. 1998). A chemical alternative for CFLP or NIRCA is the CCM assay (chemical cleavage of mismatch).

DNA sequencing methods include the Maxam and Gilbert protocol, the Sanger reaction (dideoxynucleotide chain termination reaction) and modifications thereof, pyrosequencing, cycle sequencing, SBH (sequencing by hybridization). A variation of the minisequencing procedure is GBA (Genetic Bit Analysis). These DNA sequencing methods are based on (partial) amplification of the target nucleic acid (cfr. *supra*).

Other DNA sequencing methods include molecular resonance sequencing which uses electrospray ionization (ESI) combined with Fourier transform ion cyclotron resonance (FTICR) mass spectrometry (Smith et al. 1994) and, for smaller DNA fragments, MALDI-TOF MS). Diagnostic sequencing by combining specific cleavage of DNA followed by mass spectrometric analysis of the fragments has also been described (see e.g. Stanssens and Zabeau 2000 ― WO00/66771).

In the near fixture, nanopore sequencing might also become available (Shiffman 1999).

For analyzing nucleotide sequence variation in RNA target molecules, both ribozymes (hammerhead-, hairpin-, group I intron-, ribonuclease P- or hepatitis delta viral-type ribozymes) or deoxyribozymes ('DNAzymes') can be used. This feature is moreover the basis for the possible use of these enzymes as therapeutics or in gene therapy (James and al-Shamkhani 1995; Cairns et al. 2000).

The DNA sequencing methodology known as SBH or sequencing-by-hybridization uses an array of all possible n-nucleotide oligomers (n-mers) to identify said n-mers comprised in an unknown DNA sample (Drmanac et al. 1993). Such high-density oligonucleotide arrays are useful for detecting DNA sequence variations as well, the array eventually becoming a VDA (variant detector array) (Hacia et al. 1996; Sapolsky et al. 1999). Microscope slides can be replaced by optical fibers as solid support for the oligonucleotides (Healey et al. 1997). A variation of the above-described SBH is based on solution hybridization of probes with a known information region or information tags with the target DNA fragments to be sequenced. The information tag can be a DNA bar code (eventually comprising modified bases), a molecular bar code or a nanoparticle bar code and forms the basis for identification and characterization of the hybridized target DNA (Drmanac 2000 - WO/0056937). Said high-density oligonucleotide arrays or DNA chips abolish the need to design a set of oligonucleotides specifically hybridizing under the same conditions to a set of polymorphic nucleotide sequences. The latter approach is applied in conventional reverse blot assays by carefully adjusting length, polarity and position of the mismatched nucleotide(s) in the oligonucleotide probe (Saiki et al. 1989). Conventional reverse blot hybridization assays for genotyping and detection of nucleotide sequence polymorphisms have been successfully commercialized, e.g. in the LiPA (Line Probe Assay) format (Innogenetics, Ghent, Belgium) (Stuyver et al. 1996a; Stuyver et al. 1996b; Stuyver et al. 1997).

Alternatively, Acrydite™-modified oligonucleotide probes are copolymerized into a polyacrylamide gel. Single-stranded target DNA targets are electrophoresed through said gel and, depending on electrophoresis conditions (temperature and/or denaturant), captured by the oligonucleotides immobilized in a capture gel layer. This method is also applicable for detecting nucleotide sequence polymorphisms (Kenney et al. 1998).

Other hybridization-based methods for detecting nucleotide sequence variations include the solution phase sandwich hybridization assay in which the target DNA is captured by a target-specific immobilized capture probe and detected via an amplifier or linker probe. Two methods of signal generation have been described. A first one utilizes a branched oligonucleotide hybridizing to the flap of the linker probe not binding to the target DNA. Subsequently a labeled probe is hybridized to the branches of the amplifier probe and the amount of bound label is quantified. In a second method, a (partially) double stranded amplifier probe is hybridized to the flap of the linker probe not binding to the target DNA. The double stranded (part of) said amplifier probe comprises a promoter recognized by a DNA-dependent RNA polymerase. The signal generated is formed by newly transcribed RNA from the amplifier probe, the amount of which is quantified. (see e.g. Urdea 1991 - WO91/10746).

The invention further relates to any of the methods as described above wherein said amplification is a one-step RT-PCR.

The invention further relates to any of the methods as described above wherein the HCV NS5B nucleic acid is present in a biological sample. The term "biological sample" generally refers to any biological sample (tissue or fluid) containing traces of HCV such as nucleic acid sequences. More particularly, blood serum or plasma samples are meant.

The invention also relates to a recombinant vector comprising an NS5B fragment amplified according to any of the above described methods. Said vector may be a cloning tool or may additionally comprise regulatory sequences such as promoters, enhancers and terminators or polyadenylation signals. It may also comprise any other HCV or non-HCV nucleic acid sequence, different from the amplified NS5B nucleic acid according to the invention, for example the 5' end of an HCV NS5B nucleic acid, the HCV NS3 nucleic acid, the HCV NS4 nucleic acid, an indicator gene, a reporter gene, etc. Said vector can be a "resistance test vector" as defined in *e.g.* WO 99/06597. Said vector can be an expression vector capable of directing expression of the HCV NS5B protein or any other protein encoded by the HCV nucleic acid sequence comprised in said vector. More specifically, said vector can be used for determining susceptibility or resistance of the comprised nucleic acid to an antiviral drug (e.g. WO 99/06597). Host cells transformed with a vector according to the invention are also part of the present invention and are known in the art.

In another aspect, the present invention relates to a set of primers for amplification of an HCV NS5B nucleic acid of any one of HCV genotypes 1 to 6, said set of primers characterized in that at least one of the primers is an antisense primer that is capable of annealing to the HCV NS5B genomic region spanning nucleotides 9276 to 9298, wherein the nucleotide positions correspond with the nucleotide numbering for HCV strain HCV-H.

Herein, SEQ ID Nos: 2, 4, 6, 8, 10, 12 are oligonucleotide sequences representing the above described antisense primers for amplification of an HCV NS5B nucleic acid of any one of HCV genotypes 1 to 6. More specifically,
- SEQ ID NO 2 is the oligonucleotide sequence of an antisense primer designed for HCV genotype 1, capable of annealing to the HCV NS5B genomic region from nucleotide position 9276 to 9297
- SEQ ID NO 4 is the oligonucleotide sequence of an antisense primer designed for HCV genotype 2, capable of annealing to the HCV NS5B genomic region from nucleotide position 9276 to 9298
- SEQ ID NO 6 is the oligonucleotide sequence of an antisense primer designed for HCV genotype 3, capable of annealing to the HCV NS5B genomic region from nucleotide position 9276 to 9297
- SEQ ID NO 8 is is the oligonucleotide sequence of an antisense primer designed for HCV genotype 4, capable of annealing to the HCV NS5B genomic region from nucleotide position 9276 to 9297
- SEQ ID NO 10 is the oligonucleotide sequence of an antisense primer designed for HCV genotype 5, capable of annealing to the HCV NS5B genomic region from nucleotide position 9276 to 9298
- SEQ ID NO 12 is the oligonucleotide sequence of an antisense primer designed for HCV genotype 6, capable of annealing to the HCV NS5B genomic region from nucleotide position 9276 to 9296

The invention further relates to the set of primers as described above for amplification of an HCV NS5B nucleic acid of any one of HCV genotypes 1 to 6, said set of primers further characterized in that it comprises a sense primer that is capable of annealing to the HCV NS5B genomic region spanning nucleotides 7500 to 7555, wherein the nucleotide positions correspond with the nucleotide numbering for HCV strain HCV-H.

The invention further relates to the set of primers as described above for amplification of an HCV NS5B nucleic acid from HCV genotype 1, said set of primers further characterized in that it comprises a sense primer that is capable of annealing to the HCV NS5B genomic region spanning nucleotides 7536 to 7555, wherein the nucleotide positions correspond with the nucleotide numbering for HCV strain HCV-H.

The invention further relates to the set of primers as described above for amplification of an HCV NS5B nucleic acid of any one of HCV genotypes 2 to 6, said set of primers further characterized in that it comprises a sense primer that is capable of annealing to the HCV NS5B genomic region spanning nucleotides 7500 to 7521, wherein the nucleotide positions correspond with the nucleotide numbering for HCV strain HCV-H.

Herein, SEQ ID Nos: 13, 14, 15, 16, 17, 18 are oligonucleotide sequences representing the above described sense primers for amplification of an HCV NS5B nucleic acid of any one of HCV genotypes 1 to 6. More specifically,
- SEQ ID NO 13 is the oligonucleotide sequence of a sense primer designed for HCV genotype 1, capable of annealing to the HCV NS5B genomic region from nucleotide position 7536 to 7555
- SEQ ID NO 14 is the oligonucleotide sequence of a sense primer designed for HCV genotype 2, capable of annealing to the HCV NS5B genomic region from nucleotide position 7500 to 7521
- SEQ ID NO 15 is the oligonucleotide sequence of a sense primer designed for HCV genotype 3, capable of annealing to the HCV NS5B genomic region from nucleotide position 7500 to 7517
- SEQ ID NO 16 is the oligonucleotide sequence of a sense primer designed for HCV genotype 4, capable of annealing to the HCV NS5B genomic region from nucleotide position 7500 to 7518
- SEQ ID NO 17 is the oligonucleotide sequence of a sense primer designed for HCV genotype 5, capable of annealing to the HCV NS5B genomic region from nucleotide position 7500 to 7517
- SEQ ID NO 18 is the oligonucleotide sequence of a sense primer designed for HCV genotype 6, capable of annealing to the HCV NS5B genomic region from nucleotide position 7500 to 7517

The invention further relates to the set of primers as described above for amplification of an HCV NS5B nucleic acid of any one of HCV genotypes 1 to 6, said set of primers further characterized in that it comprises a sense primer that is capable of annealing to the HCV NS5B genomic region spanning nucleotides 8258 to 8278, wherein the nucleotide positions correspond with the nucleotide numbering for HCV strain HCV-H.

Herein, SEQ ID Nos: 1, 3, 5, 7, 9, 11 are oligonucleotide sequences representing the above described sense primers for amplification of an HCV NS5B nucleic acid of any one of HCV genotypes 1 to 6. More specifically,
- SEQ ID NO 1 is the oligonucleotide sequence of a sense primer designed for HCV genotype 1, capable of annealing to the HCV NS5B genomic region from nucleotide position 8258 to 8278
- SEQ ID NO 3 is the oligonucleotide sequence of a sense primer designed for HCV genotype 2, capable of annealing to the HCV NS5B genomic region from nucleotide position 8258 to 8278
- SEQ ID NO 5 is the oligonucleotide sequence of a sense primer designed for HCV genotype 3, capable of annealing to the HCV NS5B genomic region from nucleotide position 8259 to 8278
- SEQ ID NO 7 is is the oligonucleotide sequence of a sense primer designed for HCV genotype 4, capable of annealing to the HCV NS5B genomic region from nucleotide position 8258 to 8278
- SEQ ID NO 9 is the oligonucleotide sequence of a sense primer designed for HCV genotype 5, capable of annealing to the HCV NS5B genomic region from nucleotide position 8258 to 8278
- SEQ ID NO 11 is the oligonucleotide sequence of a sense primer designed for HCV genotype 6, capable of annealing to the HCV NS5B genomic region from nucleotide position 8259 to 8278

In a further aspect, the present invention relates to a set of primers for amplification of an HCV NS5B nucleic acid of HCV genotype 1, said set of primers characterized in that at least one of the primers is an antisense primer that is capable of annealing to the HCV NS5B genomic region spanning nucleotides 8529 to 8546, wherein the nucleotide positions correspond with the nucleotide numbering for HCV strain HCV-H.

Herein, SEQ ID No: 19 is an oligonucleotide sequence representing the above described antisense primers for amplification of an HCV NS5B nucleic acid of HCV genotype 1. More specifically,
- SEQ ID NO: 19 is the oligonucleotide sequence of an antisense primer designed for HCV genotype 1, capable of annealing to the HCV NS5B genomic region from nucleotide position 8529 to 8546

The invention further relates to the set of primers as described above for amplification of an HCV NS5B nucleic acid of HCV genotype 1, said set of primers further characterized in that it comprises a sense primer that is capable of annealing to the HCV NS5B genomic region spanning nucleotides 7536 to 7555, wherein the nucleotide positions correspond with the nucleotide numbering for HCV strain HCV-H.

Herein, SEQ ID NO: 13 is an oligonucleotide sequence representing the above described sense primers for amplification of an HCV NS5B nucleic acid of HCV genotype 1. More specifically,
- SEQ ID NO 13 is the oligonucleotide sequence of a sense primer designed for HCV genotype 1, capable of annealing to the HCV NS5B genomic region from nucleotide position 7536 to 7555

The present invention also relates to a set of primers for amplification of an HCV NS5B nucleic acid of HCV genotype 1, said set of primers characterized in that at least one of the primers is a sense primer that is capable of annealing to the HCV NS5B genomic region spanning nucleotides 7536 to 7555, wherein the nucleotide positions correspond with the nucleotide numbering for HCV strain HCV-H.

The invention further relates to the set of primers as described above for amplification of an HCV NS5B nucleic acid of HCV genotype 1, said set of primers further characterized in that it comprises an antisense primer that is capable of annealing to the HCV NS5B genomic region spanning nucleotides 8529 to 8546, wherein the nucleotide positions correspond with the nucleotide numbering for HCV strain HCV-H.

In still a further aspect, the present invention relates to a set of primers for amplification of an HCV NS5B nucleic acid of HCV genotype 2, said set of primers characterized in that at least one of the primers is an antisense primer that is capable of annealing to the HCV NS5B genomic region spanning nucleotides 8620 to 8638, wherein the nucleotide positions correspond with the nucleotide numbering for HCV strain HCV-H.

Herein, SEQ ID NO: 20 is an oligonucleotide sequence representing the above described antisense primers for amplification of an HCV NS5B nucleic acid of HCV genotype 2. More specifically,
- SEQ ID NO: 20 is the oligonucleotide sequence of an antisense primer designed for HCV genotype 2, capable of annealing to the HCV NS5B genomic region from nucleotide position 8620 to 8638

The invention further relates to the set of primers as described above for amplification of an HCV NS5B nucleic acid of HCV genotype 2, said set of primers further characterized in that it comprises a sense primer that is capable of annealing to the HCV NS5B genomic region spanning nucleotides 7500 to 7521, wherein the nucleotide positions correspond with the nucleotide numbering for HCV strain HCV-H.

Herein, SEQ ID NO: 14 is an oligonucleotide sequence representing the above described sense primers for amplification of an HCV NS5B nucleic acid of HCV genotype 2. More specifically,
- SEQ ID NO: 14 is the oligonucleotide sequence of a sense primer designed for HCV genotype 2, capable of annealing to the HCV NS5B genomic region from nucleotide position 7500 to 7521

The present invention also relates to a set of primers for amplification of an HCV NS5B nucleic acid of HCV genotype 2, said set of primers characterized in that at least one of the primers is a sense primer that is capable of annealing to the HCV NS5B genomic region spanning nucleotides 7500 to 7521, wherein the nucleotide positions correspond with the nucleotide numbering for HCV strain HCV-H.

The invention further relates to the set of primers as described above for amplification of an HCV NS5B nucleic acid of HCV genotype 2, said set of primers further characterized in that it comprises an antisense primer that is capable of annealing to the HCV NS5B genomic region spanning nucleotides 8620 to 8638, wherein the nucleotide positions correspond with the nucleotide numbering for HCV strain HCV-H.

In still a further aspect, the present invention relates to a set of primers for amplification of an HCV NS5B nucleic acid of any one of HCV genotypes 3 to 5, said set of primers characterized in that at least one of the primers is an antisense primer that is capable of annealing to the HCV NS5B genomic region spanning nucleotides 8470 to 8491, wherein the nucleotide positions correspond with the nucleotide numbering for HCV strain HCV-H.

Herein, SEQ ID Nos: 21, 22, 23 are oligonucleotide sequences representing the above described antisense primers for amplification of an HCV NS5B nucleic acid of any one of HCV genotypes 3 to 5. More specifically,
- SEQ ID NO 21 is the oligonucleotide sequence of an antisense primer designed for HCV genotype 3, capable of annealing to the HCV NS5B genomic region from nucleotide position 8470 to 8491
- SEQ ID NO 22 is the oligonucleotide sequence of an antisense primer designed for HCV genotype 4, capable of annealing to the HCV NS5B genomic region from nucleotide position 8473 to 8491
- SEQ ID NO 23 is the oligonucleotide sequence of an antisense primer designed for HCV genotype 5, capable of annealing to the HCV NS5B genomic region from nucleotide position 8470 to 8488

The invention further relates to the set of primers as described above for amplification of an HCV NS5B nucleic acid of any one of HCV genotypes 3 to 5, said set of primers further characterized in that it comprises a sense primer that is capable of annealing to the HCV NS5B genomic region spanning nucleotides 7500 to 7521, wherein the nucleotide positions correspond with the nucleotide numbering for HCV strain HCV-H.

Herein, SEQ ID Nos: 15, 16, 17 are oligonucleotide sequences representing the above described sense primers for amplification of an HCV NS5B nucleic acid of any one of HCV genotypes 3 to 5. More specifically,
- SEQ ID NO 15 is the oligonucleotide sequence of a sense primer designed for HCV genotype 3, capable of annealing to the HCV NS5B genomic region from nucleotide position 7500 to 7517
- SEQ ID NO 16 is the oligonucleotide sequence of a sense primer designed for HCV genotype 4, capable of annealing to the HCV NS5B genomic region from nucleotide position 7500 to 7518
- SEQ ID NO 17 is the oligonucleotide sequence of a sense primer designed for HCV genotype 5, capable of annealing to the HCV NS5B genomic region from nucleotide position 7500 to 7517

The present invention also relates to a set of primers for amplification of an HCV NS5B nucleic acid of any one of HCV genotypes 3 to 5, said set of primers characterized in that at least one of the primers is a sense primer that is capable of annealing to the HCV NS5B genomic region spanning nucleotides 7500 to 7521, wherein the nucleotide positions correspond with the nucleotide numbering for HCV strain HCV-H.

The invention further relates to the set of primers as described above for amplification of an HCV NS5B nucleic acid of any one of HCV genotypes 3 to 5, said set of primers further characterized in that it comprises an antisense primer that is capable of annealing to the HCV NS5B genomic region spanning nucleotides 8470 to 8491, wherein the nucleotide positions correspond with the nucleotide numbering for HCV strain HCV-H.

In still a further aspect, the present invention relates to a set of primers for amplification of an HCV NS5B nucleic acid of HCV genotype 6, said set of primers characterized in that at least one of the primers is an antisense primer that is capable of annealing to the HCV NS5B genomic region spanning nucleotides 8529 to 8554, wherein the nucleotide positions correspond with the nucleotide numbering for HCV strain HCV-H.

Herein, SEQ ID NO: 24 is an oligonucleotide sequence representing the above described antisense primers for amplification of an HCV NS5B nucleic acid of HCV genotype 6. More specifically,
- SEQ ID NO: 24 is the oligonucleotide sequence of an antisense primer designed for HCV genotype 6, capable of annealing to the HCV NS5B genomic region from nucleotide position 8534 to 8554

The invention further relates to the set of primers as described above for amplification of an HCV NS5B nucleic acid of HCV genotype 6, said set of primers further characterized in that it comprises a sense primer that is capable of annealing to the HCV NS5B genomic region spanning nucleotides 7500 to 7521, wherein the nucleotide positions correspond with the nucleotide numbering for HCV strain HCV-H.

Herein, SEQ ID NO: 18 is an oligonucleotide sequence representing the above described sense primers for amplification of an HCV NS5B nucleic acid of HCV genotype 6. More specifically,
- SEQ ID NO: 18 is the oligonucleotide sequence of a sense primer designed for HCV genotype 6, capable of annealing to the HCV NS5B genomic region from nucleotide position 7500 to 7517

The present invention also relates to a set of primers for amplification of an HCV NS5B nucleic acid of HCV genotype 6, said set of primers characterized in that at least one of the primers is a sense primer that is capable of annealing to the HCV NS5B genomic region spanning nucleotides 7500 to 7521, wherein the nucleotide positions correspond with the nucleotide numbering for HCV strain HCV-H.

The invention further relates to the set of primers as described above for amplification of an HCV NS5B nucleic acid of HCV genotype 6, said set of primers further characterized in that it comprises an antisense primer that is capable of annealing to the HCV NS5B genomic region spanning nucleotides 8529 to 8554, wherein the nucleotide positions correspond with the nucleotide numbering for HCV strain HCV-H.

In still a further aspect, the invention relates to a set of primers for amplification of an HCV NS5B nucleic acid of any one of HCV genotypes 1 to 6, said set of primers characterized in that one of the primers is an antisense primer chosen from the group consisting of
- an antisense primer capable of annealing to the NS5B genomic region spanning nucleotides 9276 to 9298,
- an antisense primer capable of annealing to the NS5B genomic region spanning nucleotides 8529 to 8546,
- an antisense primer capable of annealing to the NS5B genomic region spanning nucleotides 8620 to 8638,
- an antisense primer capable of annealing to the NS5B genomic region spanning nucleotides 8470 to 8491,
- an antisense primer capable of annealing to the NS5B genomic region spanning nucleotides 8529 to 8554,
   and that the other primer is a sense primer chosen from the group consisting of
- a sense primer capable of annealing to the NS5B genomic region spanning nucleotides 7500 to 7555,
- a sense primer capable of annealing to the NS5B genomic region spanning nucleotides 7536 to 7555,
- a sense primer capable of annealing to the NS5B genomic region spanning nucleotides 7500 to 7521,
- a sense primer capable of annealing to the NS5B genomic region spanning nucleotides 8258 to 8278,
and wherein the nucleotide positions correspond with the nucleotide numbering for HCV strain HCV-H.

The antisense and sense primers according to the invention can also be used in nucleic acid sequencing reactions or as genotype-specific probes.

In another aspect, the invention relates to the use of any of the sets of primers as described above for the manufacture of a kit for amplification of an HCV NS5B nucleic acid of any one of HCV genotypes 1 to 6.

In still another aspect, the invention relates to a kit for amplification of an HCV NS5B nucleic acid of any one of HCV genotypes 1 to 6, said kit comprising any of the sets of primers as described above. Said kit can be used for multiplex amplification.

A further embodiment relates to a kit for assaying sequence variations in an HCV NS5B nucleic acid of any one of HCV genotypes 1 to 6, said kit comprising any of the sets of primers as described above. Said sequence variations can be mutations that are induced by HCV antiviral agents, mutations causing HCV drug resistance, or genotype-specific nucleotide variations.

Said kits may further comprise:
- at least one probe containing a sequence complementary to and capable of hybridizing to said amplified HCV NS5B nucleic acid according to the invention, said at least one probe serving to detect the amplified product and/or to detect sequence variations;
- a means for denaturing nucleic acids;
- an enzyme capable of modifying a double stranded or single stranded nucleic acid molecule;
- a hybridization buffer, or components necessary for producing said buffer;
- a wash solution, or components necessary for producing said solution;
- a means for detecting partially or completely denatured nucleic acids and/or a means for detecting hybrids formed in the preceding hybridization and/or a means for detecting enzymatic modifications of nucleic acids, wherein said means is producing a discriminatory signal;
- a means for attaching said at least one probe to a known location on a solid support;
- a means for infering from a detected discriminatory signal, and/or from the obtained NS5B nucleic acid according to the invention, the presence of said HCV NS5B nucleic acid, or the presence of at least one unique or genotype-specific or specific nucleotide therein, and, therefrom, the presence of said HCV virus in said biological sample and/or the genotype/subtype of said HCV virus in said biological sample;
- a means for infering from a detected discriminatory signal, and/or from the obtained NS5B nucleic acid according to the invention, the presence of said HCV NS5B nucleic acid, or the presence of at least one mutation therein, or the presence of at least one mutation therein which is a drug resistance mutation, in said HCV virus in said biological sample.

With "a means for infering the presence of at least one unique or genotype-specific or specific nucleotide" is meant any technique or method to localize and identify in a target nucleic acid sequence said genotype-specific or specific nucleotide. Said means can include a method performed manually, or performed computationally, or performed manually and/or computationally. Said means may include aligning and/or comparing an obtained nucleic acid sequence with a set of nucleic acid sequences contained within a database. Said means may furthermore include the result of the method being presented in the form of a report wherein said report can be in paper form, in electronic form or on a computer readable carrier or medium. Said means may furthermore include the searching of (nucleic acid and/or amino acid) sequence databases and/or the creation of (nucleic acid and/or amino acid) sequence alignments, the results of which may or may not be included in said report. Said means may furthermore include a device for detecting a discriminatory signal, or a kit insert or kit chart indicating how to interpret a detected discriminatory signal, or indicating where a specific discriminatory signal should appear, e.g. on a solid carrier carrying multiple oligonucleotides which can be arranged as spots, lines, dots, etc and possibly interpreting said discriminatory signal occurring on a specific location. Further examples of genotype-specific nucleotides and how to discriminate within a target HCV nucleic acid sequence are described for instance in WO 94/25601, WO 96/13590 and WO 03/020970.

With "a means for infering the presence of at least one mutation" is meant any technique or method to localize and identify in a target nucleic acid sequence said mutation. It will be clear by the man skilled in the art that said means can include a similar method as for infering the presence of at least one unique or genotype-specific or specific nucleotide (cfr. *supra*). It should also be clear by the skilled person that infering the presence of at least one genotype-specific nucleotide and infering the presence of at least one mutation and discriminating between them can occur simultaneously.

The examples of the present invention presented below are provided only for illustrative purposes and not to limit the scope of the invention.

### EXAMPLES

### EXAMPLE 1. Primers capable of amplifying the 3' end of an NS5B nucleic acid of HCV genotype 1 to 6

Sense and antisense primers were designed for amplification of the 3' end of an NS5B nucleic acid of HCV genotypes 1 to 6 within the HCV NS5B region spanning nucleotides 8258 to 8278 and nucleotides 9276 to 9298 respectively. The nucleotide positions are according to the nucleotide numbering for HCV strain HCV-H (GenBank accession no. M67463). To overcome genomic diversity, different primers were designed for each genotype. Due to the variability of the genome within a certain genotype, wobbles were sometimes included in the primer sequence. This was only done for frequently common polymorphisms that gave rise to a too low Tm when screened by the Metcalc software (Göttingen, Germany).

All designed genotype-specific primers were screened for their capability of amplifying the 3' end of an NS5B nucleic acid present in HCV-positive serum samples. Table 1 gives examples of sense and anti-sense primers per genotype that are capable of amplifying the NS5B fragment, according to the further described methodology. The efficiency of the amplification protocols was evaluated using HCV-positive samples from all major HCV genotypes covering a wide range of viral loads. This will be further illustrated by taking genotype 1 (subtype 1a or 1b) as an Example.

In a first stage, 10 HCV genotype 1 positive serum samples were evaluated with the genotype 1 specific set of sense and antisense primers (SEQ ID NO 1 and 2, respectively). Therefore, extraction of RNA from all serum samples was performed with the Qiagen QiAamp MiniElute Virus Spin kit (Venlo, The Netherlands) according to the manufacturar's instructions. Subsequently, RT-PCR was performed to amplify the targeted 3' end NS5B fragment using the Invitrogen SuperScript III

One Step RT-PCR System with Platinum Taq DNA polymerase (Merelbeke, Belgium), as further described below. This approach minimizes the possibility for introduction of contaminants into reactions between the RT and PCR steps, since the RT and PCR reactions are carried out sequentially, without opening of reaction tubes between steps. The PCR protocol was optimized by evaluating different temperatures of the RT step (50°C; 55°C; 60°C) and the annealing step (52°C; 54°C; 56°C), by testing different MgCl₂ concentrations (1,6mM; 2,0mM; 2,4mM), different primer concentrations (0,4µM-0,6µM-0,8µM) and different annealing times (30s and 60s), by testing different additives (DMSO and glycerine) at several concentrations (5%; 7,5%; 10%) and addition of wheat germ RNA. Optimal conditions in terms of sensitivity/specificity were as follows: an RT step of 30 minutes at 55°C, annealing temperature of 54°C and 1,6 mM MgCl₂. Both DMSO and Glycerine at a concentration of 7.5% can intensify the amplification signal without altering the specificity. Addition of wheat germ RNA increases the aspecificity of the PCR reaction.

Following the above optimized procedure, a one step RT-PCR protocol was developed: PCR was performed from 15 uL of RNA, starting with an RT step consisting of 30 min at 55 °C, followed by 2 min at 94 °C. Next, 40 cycles were performed with each consisting of 30 s at 94 °C, 30 s at 54 °C and 2 min at 68 °C . The extension time for the last cycle was increased to 5 min. The reactions were carried out with 2 µL SuperScript III RT / Platinum Taq polymerase mix in 25 uL 2X reaction mix (containing 200 µM dNTP and 1.6 mM MgCl₂) and 5.5 µL RNAse free water, in the presence of 2.5 µl primer mix (0.5 µM, containing 10 pmol/µL of each primer). The PCR products were electrophoresed in 2% agarose gel and fragments were detected by ethidium bromide staining and UV illumination.

In a second stage, the amplification efficiency of the developed one-step RT-PCR protocol was confirmed by evaluating 18 HCV genotype 1 positive serum samples with the genotype 1-specific set of sense and antisense primers (SEQ ID NO 1 and 2, respectively). The results are summarized in Table 2. The expected sizes of NS5B cDNA bands of about 1000 bp were detected in all serum samples. As such, the selected pairs of primers with SEQ ID NO 1 and SEQ ID NO 2 designed for genotype 1 within the above described regions are widely effective for amplification of the targeted NS5B region.

The same methodology as described above was used for amplification of the targeted 3' end NS5B fragment of HCV genotypes 2 to 6. It was demonstrated that the selected pairs of primers with SEQ ID NO 3 and SEQ ID NO 4 (genotype 2), SEQ ID NO 5 and SEQ ID NO 6 (genotype 3), SEQ ID NO 7 and SEQ ID NO 8 (genotype 4), SEQ ID NO 9 and SEQ ID NO 10 (genotype 5), SEQ ID NO 11 and SEQ ID NO 12 (genotype 6), designed within the two defined NS5B regions, are widely effective for amplification of the targeted NS5B region of the respective HCV genotypes 2 to 6. Briefly, 21 HCV genotype 2, 26 HCV genotype 3, 20 HCV genotype 4, 13 HCV genotype 5, 12 HCV genotype 6 positive serum samples were evaluated following the one-step RT-PCR protocol as described above under exactly the same conditions, except changing the set of primers per genotype (see Table 1). The results are summarized in Tables 3 to 7. The expected sizes of NS5B cDNA bands of about 1000 bp were detected in respectively all HCV genotype 2-6 positive serum samples.

In conclusion, the described methodology provides a standardized and rapid amplification system for the targeted 3' end of an HCV NS5B nucleic acid fragment of any of HCV genotypes 1 to 6. From Table 1 it can moreover be derived that all genotype-specific sense primers locate in the same HCV NS5B region spanning nucleotides 8258 to 8278 and similarly, that all genotype-specific antisense primers locate in the same HCV NS5B region spanning nucleotides 9276 to 9298. As such, these two defined HCV NS5B regions are universal regions wherein any primer according to genotype can be designed capable of amplification of the NS5B fragment.

**Table 1.**

| Genotype | SEQ ID NO | Sense or antisense | Position* | 5'-3' sequence |
|---|---|---|---|---|
| 1 | 1 | Sense | 8258-8278 | TGAYACCCGCTGYTTTGACTC |
| | 2 | Antisense | 9276-9297 | GAGACASGCTGTGATAWATGTC |
| 2 | 3 | Sense | 8258-8278 | TGAYACCCGMTGCTTTGACTC |
| | 4 | Antisense | 9276-9298 | CGCGACACGCTGTGATAAATGTC |
| 3 | 5 | Sense | 8259-8278 | GACACYCGYTGCTTTGACTC |
| | 6 | Antisense | 9276-9297 | GYGACACGCTGTGATAAATGTC |
| 4 | 7 | Sense | 8258-8278 | TGATACCCGCTGCTTTGACTC |
| | 8 | Antisense | 9276-9297 | GAGACATGCTGTGATAAATGTC |
| 5 | 9 | Sense | 8258-8278 | TGAYACCCGCTGCTTTGACTC |
| | 10 | Antisense | 9276-9298 | CGGGACATGCTGTGATAAATGTC |
| 6 | 11 | Sense | 8259-8278 | GACACCAGRTGYTTTGATTC |
| | 12 | Antisense | 9276-9296 | GGACACGCTGTGATAAATGTC |

| | | | | |
|---|---|---|---|---|
| *The nucleotide positions correspond with the nucleotide numbering of HCV strain HCV-H (GenBank accession no. M67463). Y is T or C ; S is G or C; M is A or C; W is A or T; K is G or T; R is G or A | | | | |

**Table 2.**

| Sample | Genotype | Viral Load (cp/ml) | NS5B fragment amplified with primer pair SEQ ID NO 1/SEQ ID NO 2 |
|---|---|---|---|
| 248-085 | 1a | 74.723 | ++ |
| 248-020 | 1a | 108.759 | + |
| 248-049 | 1a | 311.743 | ++ |
| 242-008 | 1a | 502.970 | ++ |
| 248-055 | 1a | 993.776 | ++ |
| 248-036 | 1a | 1.073.451 | + |
| 248-042 | 1a | 5.594.083 | ++ |
| 242-031 | 1a | 13.685.001 | ++ |
| 242-029 | 1b | 54.564 | ++ |
| 242-049 | 1b | 855.738 | +++ |
| 248-019 | 1b | 1.096.434 | ++ |
| 248-048 | 1b | 1.152.486 | ++ |
| 235-019 | 1b | 1.341.527 | +++ |
| 248-041 | 1b | 3.024.771 | ++ |
| 248-031 | 1b | 3.616.082 | ++ |
| 235-015 | 1b | 8.317.655 | ++ |
| 248-115 | 1b | 8.317.655 | ++ |
| 235-006 | 1b | 10.891.026 | ++ |

| | | | |
|---|---|---|---|
| +, ++, +++ : amplification product with increasing signal intensity, respectively | | | |

**Table 3**

| Sample | Genotype | Viral Load | NS5B fragment amplified with primer pair SEQ ID NO 3/SEQ ID NO 4 |
|---|---|---|---|
| 242-004 | 2a | 243.714 | +++ |
| 242-002 | 2a | 403.884 | +++ |
| 242-019 | 2a | 5.542.456 | +++ |
| IG57123 | 2a | n.a. | + |
| IG57131 | 2a | n.a. | + |
| IG57171 | 2a, 2c | n.a. | ++ |
| IG57193 | 2a, 2c | n.a. | ++ |
| IG57208 | 2a, 2c | n.a. | ++ |
| IG94409 | 2a, 2c | n.a. | + |
| IG89689 | 2 | 1.420.000 | ++ |
| IG93818 | 2b | 1.742.000 | + |
| IG89678 | 2b | 2.600.000 | ++ |
| INNO3023 | 2 | 2.860.000 | ++ |
| INNO3022 | 2 | 5.200.000 | ++ |
| IG57012 | 2 | n.a. | ++ |
| IG57027 | 2b | n.a. | ++ |
| IG57059 | 2 | n.a. | + |
| IG57205 | 2 | n.a. | ++ |
| IG93870 | 2b | n.a. | ++ |
| IG93871 | 2b | n.a. | ++ |
| 235-021 | 2c | 10.593.768 | +++ |

| | | | |
|---|---|---|---|
| +, ++, +++ : amplification product with increasing signal intensity, respectively | | | |

**Table 4**

| Sample | Genotype | Viral Load | NS5B fragment amplified with primer pair SEQ ID NO 5/SEQ ID NO 6 |
|---|---|---|---|
| 248-137 | 3a | 100.599 | ++ |
| IG93743 | 3a | 119.080 | + |
| IG93851 | 3a | 257.400 | + |
| 235-008 | 3a | 538.866 | ++ |
| 235-014 | 3a | 850.881 | ++ |
| IG89658 | 3a | 1.080.000 | +++ |
| IG92572 | 3a | 1.482.000 | ++ |
| INNO3026 | 3 | 1.852.646 | +++ |
| 248-034 | 3a | 1.871.533 | ++ |
| IG93711 | 3a | 3.244.800 | +++ |
| IG93551 | 3 | 5.028.400 | +++ |
| 235-011 | 3a | 15.895.142 | ++ |
| 235-012 | 3a | 27.320.368 | +++ |
| IG57160 | 3 | n.a. | ++ |
| IG57166 | 3a | n.a. | ++ |
| IG57180 | 3a | n.a. | ++ |
| IG57186 | 3a | n.a. | ++ |
| IG57191 | 3 | n.a. | ++ |
| IG57222 | 3 | n.a. | ++ |
| IG57105 | 3a | n.a. | ++ |
| IG57025 | 3a | n.a. | ++ |
| IG94407 | 3 | n.a. | +++ |
| IG94408 | 3 | n.a. | +++ |
| IG94413 | 3 | n.a. | +++ |
| IG94415 | 3 | n.a. | ++ |
| IG57007 | 3b | n.a. | ++ |

| | | | |
|---|---|---|---|
| +, ++, +++ : amplification product with increasing signal intensity, respectively | | | |

**Table 5**

| Sample | Genotype | Viral Load | NS5B fragment amplified with primer pair SEQ ID NO 7/SEQ ID NO 8 |
|---|---|---|---|
| IG93875 | 4c, 4d | 198.640 | ++ |
| IG93880 | 4c, 4d | 341.120 | + |
| IG93876 | 4c, 4d | 774.800 | + |
| IG92577 | 4a | 1.138.800 | ++ |
| IG93874 | 4c, 4d | 1.482.000 | ++ |
| 235-004 | 4d | 2.622.438 | ++ |
| INNO3032 | 4 | 3.739.715 | ++ |
| IG93575 | 4 | 5.356.000 | +++ |
| IG57162 | 4a | n.a. | + |
| IG57204 | 4a | n.a. | ++ |
| IG57091 | 4 | n.a. | + |
| IG57109 | 4 | n.a. | ++ |
| IG56999 | 4 | n.a. | + |
| IG93298 | 4a | n.a. | ++ |
| IG92993 | 4 | n.a. | +++ |
| IG92999 | 4 | n.a. | +++ |
| IG93005 | 4 | n.a. | ++ |
| IG94403 | 4 | n.a. | ++ |
| IG94404 | 4a | n.a. | ++ |
| 235-009 | 4b | 927.035 | + |

| | | | |
|---|---|---|---|
| +, ++, +++ : amplification product with increasing signal intensity, respectively | | | |

**Table 6**

| Sample | Genotype | Viral Load | NS5B fragment amplified with primer pair SEQ ID NO 9/SEQ ID NO 10 |
|---|---|---|---|
| 235-020 | 5a | 2.204.914 | ++ |
| IG92553 | 5a | 2.332.990 | ++ |
| IG92548 | 5a | 2.600.000 | +++ |
| IG92579 | 5a | 3.525.600 | ++ |
| 235-003 | 5a | 3.988.707 | ++ |
| IG92546 | 5a | 4.901.276 | +++ |
| IG92550 | 5a | 7.061.980 | +++ |
| IG92554 | 5 | 8.554.286 | ++ |
| IG92549 | 5a | 10.420.108 | +++ |
| 235-013 | 5a | 15.852.242 | ++ |
| IG92547 | 5 | n.a. | +++ |
| IG92551 | 5a | n.a. | ++ |
| IG92552 | 5a | n.a. | ++ |

| | | | |
|---|---|---|---|
| +, ++, +++ : amplification product with increasing signal intensity, respectively | | | |

**Table 7**

| Sample | Genotype | Viral Load | NS5B fragment amplified with primer pair SEQ ID NO 11/SEQ ID NO 12 |
|---|---|---|---|
| 242-035 | 6a | 18.294 | ++ |
| 242-005 | 6a | 113.646 | + |
| 242-007 | 6a | 529.490 | ++ |
| 242-044 | 6a | 543.967 | ++ |
| 242-051 | 6a | 629.086 | +++ |
| 242-001 | 6a | 715.380 | +++ |
| 242-010 | 6a | 1.474.840 | ++ |
| 242-012 | 6a | 1.614.475 | ++ |
| 242-052 | 6a | 2.106.031 | +++ |
| 247-004 | 6a | 2.225.990 | +++ |
| 242-016 | 6a | 6.515.876 | ++ |
| IG94414 | 6b | n.a. | +++ |

| | | | |
|---|---|---|---|
| +, ++, +++ : amplification product with increasing signal intensity, respectively | | | |

### EXAMPLE 2. Sensitivity of the method for amplification of the 3' end of an NS5B nucleic acid of HCV genotype 1

A dilution serie was made of 2 serum samples 242-029 (genotype 1b-positive) and 248- 085 (genotype 1a-positive). Each diluted sample was evaluated as a template for amplification of the targeted 3' end NS5B fragment using the genotype 1 specific set of sense and antisense primers in a RT-PCR reaction as described in Example 1. The results are shown in Table 8. The methodology outlined in Example 1 thus appears to be robust and works at HCV viral loads as low as 3500 to 4500 cp/mL. As such, the described methodology thus provides a highly sensitive and specific amplification system for the targeted 3' end HCV NS5B region.

### EXAMPLE 3. Sequencing of the 3' end NS5B amplified product derived from HCV positive serum samples

The designed sense and antisense primers according to HCV genotypes 1 to 6 as described in Example 1 (see Table 1) were succesfully used as sequencing primers to obtain complete coverage of both strands of the resulting amplified NS5B fragment of each genotype. As expected, the nucleic acid sequence of the amplified NS5B fragments derived from the above-mentioned HCV-positive serum samples about 1000 bp was confirmed. The present invention thus provides a uniform and rapid RT-PCR based tool for amplification and sequencing of the 3' end of an NS5B nucleic acid fragment of any one of HCV genotypes 1 to 6.

**Table 8.**

| Sample | Genotype | Viral Load (cp/ml) | Primer Pair: SEQ ID NO 1/SEQ ID NO 2 |
|---|---|---|---|
| 242-029 | 1b | 54.564 | ++ |
| 242-029 / DD16883 | 1b | 27.282 | ++ |
| 242-029 / DD16884 | 1b | 13.641 | ++ |
| 242-029 / DD16885 | 1b | 6.820,50 | + |
| 242-029 / DD16891 | 1b | 5.464,4 | ++ |
| 242-029 / DD16886 | 1b | 3.410,25 | + |
| 242-029 / DD16887 | 1b | 1.705,13 | - |
| 242-029 / DD16888 | 1b | 852,56 | - |
| 242-029 / DD16889 | 1b | 426,28 | - |
| 242-029 / DD16890 | 1b | 213,14 | - |
| 248-085 | 1a | 74.723 | ++ |
| 248-085 / DD16892 | 1a | 37.361,5 | ++ |
| 248-085 / DD16893 | 1a | 18.680,75 | ++ |
| 248-085 / DD16894 | 1a | 9.340,38 | + |
| 248-085 / DD16895 | 1a | 4.670,19 | + |
| 248-085 / DD16896 | 1a | 2.335,09 | - |
| 248-085 / DD16897 | 1a | 1.167,55 | + |
| 248-085 / DD16898 | 1a | 583,77 | - |
| 248-085 / DD16899 | 1a | 291,89 | - |
| 248-085 / DD16900 | 1a | 145,9 | - |

| | | | |
|---|---|---|---|
| - : no amplification product; +, ++ : amplification product with increasing signal intensity, respectively | | | |

### EXAMPLE 4. Primers capable of amplifying the full length of an NS5B nucleic acid of HCV genotype 1 to 6

The same methodology as described in Example 1 was shown to be applicable for amplification of the entire NS5B nucleic acid of HCV genotypes 1 to 6, except that sense and antisense primers were now designed for amplification of the full length of the NS5B nucleic acid of HCV genotypes 1 to 6 within the HCV NS5B region spanning nucleotides 7500 to 7555 and nucleotides 9276 to 9298 respectively. The nucleotide positions are according to the nucleotide numbering for HCV strain HCV-H (GenBank accession no. M67463).

All designed genotype-specific primers were screened for their capability of amplifying the full length of an NS5B nucleic acid present in HCV-positive serum samples. Table 9 gives examples of sense and anti-sense primers per genotype that are capable of amplifying the entire NS5B nucleic acid, according to the methodology described in Example 1.

It was demonstrated that the selected pairs of primers with SEQ ID NO 13 and SEQ ID NO 2 (genotype 1), SEQ ID NO 14 and SEQ ID NO 4 (genotype 2), SEQ ID NO 15 and SEQ ID NO 6 (genotype 3), SEQ ID NO 16 and SEQ ID NO 8 (genotype 4), SEQ ID NO 17 and SEQ ID NO 10 (genotype 5), SEQ ID NO 18 and SEQ ID NO 12 (genotype 6), designed within the two defined NS5B regions, are widely effective for amplification of the targeted NS5B region of the respective HCV genotypes 1 to 6. Briefly, 4 HCV genotype 1, 4 HCV genotype 2, 3 HCV genotype 3, 2 HCV genotype 4, 3 HCV genotype 5, 2 HCV genotype 6 positive serum samples were evaluated following the one-step RT-PCR protocol as described in Example 1 under exactly the same conditions, except for 3 min (instead of 2 min) at 68 °C and changing the set of primers according to the genotype (see Table 9). The results are summarized in Table 10. The expected sizes of NS5B cDNA bands of about 1800 bp were detected in respectively all HCV genotype 1-6 positive serum samples.

In conclusion, the described methodology provides a standardized and rapid amplification system for the targeted full length of an HCV NS5B nucleic acid fragment of any of HCV genotypes 1 to 6. From Table 9 it can moreover be derived that all genotype-specific sense primers locate in the same HCV NS5B region spanning nucleotides 7500 to 7555 and similarly, that all genotype-specific antisense primers locate in the same HCV NS5B region spanning nucleotides 9276 to 9298. As such, these two defined HCV NS5B regions are universal regions wherein any primer according to genotype can be designed capable of amplification of the entire NS5B nucleic acid.

**Table 9.**

| Genotype | SEQ ID NO | Sense or antisense | Position* | 5'-3' sequence |
|---|---|---|---|---|
| 1 | 13 | Sense | 7536-7555 | GATCTCAGYGACGGGTCWTG |
| | 2 | Antisense | 9276-9297 | GAGACASGCTGTGATAWATGTC |
| 2 | 14 | Sense | 7500-7521 | GCTCCATGTCATACTCCTGGAC |
| | 4 | Antisense | 9276-9298 | CGCGACACGCTGTGATAAATGTC |
| 3 | 15 | Sense | 7500-7517 | TCCATGCCKCCTCTYGAG |
| | 6 | Antisense | 9276-9297 | GYGACACGCTGTGATAAATGTC |
| 4 | 16 | Sense | 7500-7518 | CGTCAATGCCTCCACTAGAG |
| | 8 | Antisense | 9276-9297 | GAGACATGCTGTGATAAATGTC |
| 5 | 17 | Sense | 7500-7517 | TCCATGCCTCCGCTRGAG |
| | 10 | Antisense | 9276-9298 | CGGGACATGCTGTGATAAATGTC |
| 6 | 18 | Sense | 7500-7517 | TCAATGCCCCCYCTYGAG |
| | 12 | Antisense | 9276-9296 | GGACACGCTGTGATAAATGTC |

| | | | | |
|---|---|---|---|---|
| *The nucleotide positions correspond with the nucleotide numbering HCV strain HCV-H (GenBank accession no. M67463). Y is T or C ; S is G or C; W is A or T; K is G or T; R is G or A | | | | |

**Table 10**

| Sample | Genotype | Viral Load (cp/ml) | Primer Pair |
|---|---|---|---|
| | | | SEQ ID NO 13/SEQ ID NO 2 |
| 248-042 | 1a | 5.594.083 | + |
| 242-031 | 1a | 13.685.001 | + |
| 235-019 | 1b | 1.341.527 | + |
| 248-115 | 1b | 8.317.655 | + |

| | | | SEQ ID NO 14/SEQ ID NO 4 |
|---|---|---|---|
| 242-019 | 2a | 5.542.456 | ++ |
| INNO3023 | 2 | 2.860.000 | + |
| INNO3022 | 2 | 5.200.000 | + |
| 235-021 | 2c | 10.593.768 | ++ |

| | | | SEQ ID NO 15/SEQ ID NO 6 |
|---|---|---|---|
| INNO3026 | 3 | 1.852.646 | ++ |
| IG93551 | 3 | 5.028.400 | ++ |
| 235-011 | 3a | 15.895.142 | ++ |

| | | | SEQ ID NO 16/SEQ ID NO 8 |
|---|---|---|---|
| IG92577 | 4a | 1.138.800 | +++ |
| 235-004 | 4d | 2.622.438 | + |

| | | | SEQ ID NO 17/SEQ ID NO 10 |
|---|---|---|---|
| 235-020 | 5a | 2.204.914 | + |
| 235-003 | 5a | 3.988.707 | + |
| 235-013 | 5a | 15.852.242 | + |

| | | | SEQ ID NO 18/SEQ ID NO 12 |
|---|---|---|---|
| 242-016 | 6a | 6.515.876 | + |
| IG94414 | 6b | n.a. | + |

| | | | |
|---|---|---|---|
| +, ++, +++ : amplification product with increasing signal intensity, respectively | | | |

### EXAMPLE 5. Primers capable of amplifying the 5' end of an NS5B nucleic acid of HCV genotype 1

Sense and antisense primers were designed for amplification of the 5' end of an NS5B nucleic acid of HCV genotype 1 within the HCV NS5B region spanning nucleotides 7536 to 7555 and nucleotides 8529 to 8546 respectively. The nucleotide positions are according to the nucleotide numbering for HCV strain HCV-H (GenBank accession no. M67463). Due to the variability of the genome within a genotype, wobbles were sometimes included in the primer sequence. This was only done for frequently common polymorphisms that gave rise to a too low Tm when screened by the Metcalc software (Göttingen, Germany).

All designed genotype-specific primers were screened for their capability of amplifying the 5' end of an NS5B nucleic acid present in HCV-positive serum samples. Table 11 gives examples of sense and anti-sense primers for genotype 1 that are capable of amplifying the NS5B fragment, according to the further described methodology. The efficiency of the amplification protocols was evaluated using HCV-positive samples of HCV genotype 1 covering a wide range of viral loads.

In a first stage, 10 HCV genotype 1 positive serum samples were evaluated with the genotype 1 specific set of sense and antisense primers (SEQ ID NO 13 and 19, respectively). Therefore, extraction of RNA from all serum samples was performed with the Qiagen QiAamp MiniElute Virus Spin kit (Venlo, The Netherlands) according to the manufacturar's instructions. Subsequently, RT-PCR was performed to amplify the targeted 5' end NS5B fragment using the Invitrogen SuperScript III One Step RT-PCR System with Platinum Taq DNA polymerase (Merelbeke, Belgium), as further described below. This approach minimizes the possibility for introduction of contaminants into reactions between the RT and PCR steps, since the RT and PCR reactions are carried out sequentially, without opening of reaction tubes between steps. The PCR protocol was optimized by evaluating different temperatures of the RT step (50°C; 55°C; 60°C) and the annealing step (52°C; 54°C; 56°C), by testing different MgCl₂ concentrations (1,6mM; 2,0mM; 2,4mM), different primer concentrations (0,4µM-0,6µM-0,8µM) and different annealing times (30s and 60s), by testing different additives (DMSO and glycerine) at several concentrations (5%; 7,5%; 10%) and addition of wheat germ RNA. Optimal conditions in terms of sensitivity/specificity were as follows: an RT step of 30 minutes at 55°C, annealing temperature of 54°C and 1,6 mM MgCl₂. Both DMSO and Glycerine at a concentration of 7.5% can intensify the amplification signal without altering the specificity. Addition of wheat germ RNA increases the aspecificity of the PCR reaction.

Following the above optimized procedure, a one step RT-PCR protocol was developed: PCR was performed from 15 uL of RNA, starting with an RT step consisting of 30 min at 55 °C, followed by 2 min at 94 °C. Next, 40 cycles were performed with each consisting of 30 s at 94 °C, 30 s at 54 °C and 2 min at 68 °C . The extension time for the last cycle was increased to 5 min. The reactions were carried out with 2 µL SuperScript III RT / Platinum Taq polymerase mix in 25 uL 2X reaction mix (containing 200 µM dNTP and 1.6 mM MgCl₂) and 5.5 µL RNAse free water, in the presence of 2.5 µl primer mix (0.5 µM, containing 10 pmol/µL of each primer). The PCR products were electrophoresed in 2% agarose gel and fragments were detected by ethidium bromide staining and UV illumination.

In a second stage, the amplification efficiency of the developed one-step RT-PCR protocol was confirmed by evaluating 21 HCV genotype 1 positive serum samples with the genotype 1-specific set of sense and antisense primers (SEQ ID NO 13 and 19, respectively). The results are summarized in Table 12. The expected sizes of NS5B cDNA bands of about 1050 bp were detected in all serum samples. As such, the selected pairs of primers with SEQ ID NO 13 and SEQ ID NO 19 designed for genotype 1 within the above described regions are widely effective for amplification of the targeted NS5B region.

In conclusion, the described methodology provides a standardized and rapid amplification system for the targeted 5' end of an HCV NS5B nucleic acid fragment of HCV genotype 1. The two defined HCV NS5B regions are universal regions wherein any primer according to genotype 1 can be designed capable of amplification of the NS5B fragment.

**Table 11.**

| Genotype | SEQ ID NO | Sense or antisense | Position* | 5'-3' sequence |
|---|---|---|---|---|
| 1 | 13 | Sense | 7536-7555 | GATCTCAGYGACGGGTCWTG |
| | 19 | Antisense | 8529-8546 | CACGAGCATSGTGCAGTC |

| | | | | |
|---|---|---|---|---|
| *The nucleotide positions correspond with the nucleotide numbering of HCV strain HCV-H (GenBank accession no. M67463). Y is T or C ; S is G or C; W is A or T | | | | |

**Table 12.**

| Sample | Genotype | Viral Load (cp/ml) | NS5B fragment amplified with primer pair SEQ ID NO 13/SEQ ID NO 19 |
|---|---|---|---|
| 248-122 | 1a | 523 | ++ |
| 248-003 | 1a | 11.301 | ++ |
| 248-085 | 1a | 74.723 | ++ |
| 248-020 | 1a | 108.759 | ++ |
| 248-049 | 1a | 311.743 | ++ |
| 242-008 | 1a | 502.970 | ++ |
| 248-055 | 1a | 993.776 | +++ |
| 248-036 | 1a | 1.073.451 | ++ |
| 248-042 | 1a | 5.594.083 | ++ |
| 242-031 | 1a | 13.685.001 | +++ |
| 242-090 | 1b | 3.960 | ++ |
| 242-029 | 1b | 54.564 | ++ |
| 242-049 | 1b | 855.738 | ++ |
| 248-019 | 1b | 1.096.434 | ++ |
| 248-048 | 1b | 1.152.486 | ++ |
| 235-019 | 1b | 1.341.527 | ++ |
| 248-041 | 1b | 3.024.771 | +++ |
| 248-031 | 1b | 3.616.082 | +++ |
| 235-015 | 1b | 8.317.655 | ++ |
| 248-115 | 1b | 8.317.655 | ++ |
| 235-006 | 1b | 10.891.026 | ++ |

| | | | |
|---|---|---|---|
| +, ++, +++: amplification product with increasing signal intensity, respectively | | | |

### EXAMPLE 6. Sensitivity of the method for amplification of the 5' end of an NS5B nucleic acid of HCV genotype 1

A dilution serie was made of 2 serum samples 242-029 (genotype 1b-positive) and 248- 085 (genotype 1a-positive). Each diluted sample was evaluated as a template for amplification of the targeted 5' end NS5B fragment using the genotype 1 specific set of sense and antisense primers in a RT-PCR reaction as described in Example 5. The results are shown in Table 13. The methodology outlined in Example 5 thus appears to be robust and works at HCV viral loads as low as 1000 cp/mL. As such, the described methodology thus provides a highly sensitive and specific amplification system for the targeted 5' end HCV NS5B region.

### EXAMPLE 7. Sequencing of the 5' end NS5B amplified product derived from HCV genotype 1 positive serum samples

The designed sense and antisense primers according to HCV genotype 1 as described in Example 5 (see Table 11) were succesfully used as sequencing primers to obtain complete coverage of both strands of the resulting amplified NS5B fragment of genotype 1. As expected, the nucleic acid sequence of the amplified NS5B fragments derived from the above-mentioned HCV-positive serum samples of about 1000 bp was confirmed. The present invention thus provides a uniform and rapid RT-PCR based tool for amplification and sequencing of the 5' end of an NS5B nucleic acid fragment of HCV genotype 1.

**Table 13.**

| Sample | Genotype | Viral Load (cp/ml) | Primer Pair: SEQ ID NO 13/SEQ ID NO 19 |
|---|---|---|---|
| 242-029 | 1b | 54.564 | +++ |
| 242-029 / DD16883 | 1b | 27.282 | +++ |
| 242-029 / DD16884 | 1b | 13.641 | +++ |
| 242-029 / DD 16885 | 1b | 6.820,50 | ++ |
| 242-029 / DD16891 | 1b | 5.464,4 | +++ |
| 242-029 / DD16886 | 1b | 3.410,25 | ++ |
| 242-029 / DD16887 | 1b | 1.705,13 | + |
| 242-029 / DD16888 | 1b | 852,56 | + |
| 242-029 / DD 16889 | 1b | 426,28 | - |
| 242-029 / DD16890 | 1b | 213,14 | - |
| 248-085 | 1a | 74.723 | ++ |
| 248-085 / DD16892 | 1a | 37.361,5 | ++ |
| 248-085 / DD16893 | 1a | 18.680,75 | + |
| 248-085 / DD16894 | 1a | 9.340,38 | + |
| 248-085 / DD16895 | 1a | 4.670,19 | + |
| 248-085 / DD16896 | 1a | 2.335,09 | + |
| 248-085 / DD16897 | 1a | 1.167,55 | + |
| 248-085 / DD16898 | 1a | 583,77 | + |
| 248-085 / DD16899 | 1a | 291,89 | - |
| 248-085 / DD16900 | 1a | 145,9 | - |

| | | | |
|---|---|---|---|
| - : no amplification product; +, ++, +++ : amplification product with increasing signal intensity, respectively | | | |

### EXAMPLE 8. Primers capable of amplifying the 5' end of an NS5B nucleic acid of HCV genotype 2

Sense and antisense primers were designed for amplification of the 5' end of an NS5B nucleic acid of HCV genotype 2 within the HCV NS5B region spanning nucleotides 7500 to 7521 and nucleotides 8620 to 8638 respectively. The nucleotide positions are according to the nucleotide numbering for HCV strain HCV-H (GenBank accession no. M67463). Due to the variability of the genome within a genotype, wobbles were sometimes included in the primer sequence. This was only done for frequently common polymorphisms that gave rise to a too low Tm when screened by the Metcalc software (Göttingen, Germany).

All designed genotype-specific primers were screened for their capability of amplifying the 5' end of an NS5B nucleic acid present in HCV-positive serum samples. Table 14 gives examples of sense and anti-sense primers for genotype 2 that are capable of amplifying the NS5B fragment, according to the further described methodology. The efficiency of the amplification protocols was evaluated using HCV-positive samples of HCV genotype 2 covering a wide range of viral loads.

It was demonstrated that the selected pairs of primers with SEQ ID NO 14 and SEQ ID NO 20, designed within the two defined NS5B regions, are widely effective for amplification of the targeted NS5B region of HCV genotype 2. 21 HCV genotype 2 positive serum samples were evaluated following a one-step RT-PCR protocol as further described under exactly the same conditions. The results are summarized in Table 15. The expected sizes of NS5B cDNA bands of about 1000 bp were detected in all HCV genotype 2 positive serum samples.

Hereto, extraction of RNA from all serum samples was performed with the Qiagen QiAamp MiniElute Virus Spin kit (Venlo, The Netherlands) according to the manufacturar's instructions. Subsequently, RT-PCR was performed to amplify the targeted 5' end NS5B fragment using the Invitrogen SuperScript III One Step RT-PCR System with Platinum Taq DNA polymerase (Merelbeke, Belgium), as further described below. This approach minimizes the possibility for introduction of contaminants into reactions between the RT and PCR steps, since the RT and PCR reactions are carried out sequentially, without opening of reaction tubes between steps. The following one step RT-PCR protocol was developed: PCR was performed from 15 uL of RNA, starting with an RT step consisting of 30 min at 55 °C, followed by 2 min at 94 °C. Next, 40 cycles were performed with each consisting of 30 s at 94 °C, 30 s at 54 °C and 2 min at 68 °C . The extension time for the last cycle was increased to 5 min. The reactions were carried out with 2 µL SuperScript III RT /Platinum Taq polymerase mix in 25 uL 2X reaction mix (containing 200 µM dNTP and 1.6 mM MgCl₂) and 5.5 µL RNAse free water, in the presence of 2.5 µl primer mix (0.5 µM, containing 10 pmol/µL of each primer). The PCR products were electrophoresed in 2% agarose gel and fragments were detected by ethidium bromide staining and UV illumination.

In conclusion, the described methodology provides a standardized and rapid amplification system for the targeted 5' end of an HCV NS5B nucleic acid fragment of HCV genotype 2. The two defined HCV NS5B regions are universal regions wherein any primer according to genotype 2 can be designed capable of amplification of the NS5B fragment.

### EXAMPLE 9. Sequencing of the NS5B amplified product derived from HCV genotype 2 positive serum samples

The designed sense and antisense primers according to HCV genotype 2 as described in Example 8 (see Table 14) were succesfully used as sequencing primers to obtain complete coverage of both strands of the resulting amplified NS5B fragment of genotype 2. As expected, the nucleic acid sequence of the amplified NS5B fragments derived from the above-mentioned HCV-positive serum samples of about 1000 bp was confirmed. The present invention thus provides a uniform and rapid RT-PCR based tool for amplification and sequencing of the 5' end of an NS5B nucleic acid fragment of HCV genotype 2.

**Table 14.**

| Genotype | SEQ ID NO | Sense or antisense | Position* | 5'-3' sequence |
|---|---|---|---|---|
| 2 | 14 | Sense | 7500-7521 | GCTCCATGTCATACTCCTGGAC |
| | 20 | Antisense | 8620-8638 | TACCTGGTCATAGCCTCCG |

| | | | | |
|---|---|---|---|---|
| *The nucleotide positions correspond with the nucleotide numbering of HCV strain HCV-H (GenBank accession no. M67463). | | | | |

**Table 15**

| Sample | Genotype | Viral Load | NS5B fragment amplified with primer pair SEQ ID NO 14/SEQ ID NO 20 |
|---|---|---|---|
| 242-004 | 2a | 243.714 | + |
| 242-002 | 2a | 403.884 | ++ |
| 242-019 | 2a | 5.542.456 | +++ |
| IG57123 | 2a | n.a. | ++ |
| IG57131 | 2a | n.a. | + |
| IG57171 | 2a, 2c | n.a. | + |
| IG57193 | 2a, 2c | n.a. | ++ |
| IG57208 | 2a, 2c | n.a. | +++ |
| IG89689 | 2 | 1.420.000 | ++ |
| IG93818 | 2b | 1.742.000 | ++ |
| IG93822 | 2b | 2.459.600 | + |
| IG89678 | 2b | 2.600.000 | ++ |
| INNO3023 | 2 | 2.860.000 | ++ |
| INNO3022 | 2 | 5.200.000 | ++ |
| IG57012 | 2 | n.a. | ++ |
| IG57044 | 2b | n.a. | + |
| IG57059 | 2 | n.a. | ++ |
| IG57205 | 2 | n.a. | ++ |
| IG93870 | 2b | n.a. | +++ |
| IG93871 | 2b | n.a. | +++ |

| | | | |
|---|---|---|---|
| +, ++, +++ : amplification product with increasing signal intensity, respectively | | | |

### EXAMPLE 10. Primers capable of amplifying the 5' end of an NS5B nucleic acid of HCV genotypes 3 to 5

Sense and antisense primers were designed for amplification of the 5' end of an NS5B nucleic acid of HCV genotypes 3 to 5 within the HCV NS5B region spanning nucleotides 7500 to 7521 and nucleotides 8470 to 8491 respectively. The nucleotide positions are according to the nucleotide numbering for HCV strain HCV-H (GenBank accession no. M67463). To overcome genomic diversity, different primers were designed for each genotype. Due to the variability of the genome within a certain genotype, wobbles were sometimes included in the primer sequence. This was only done for frequently common polymorphisms that gave rise to a too low Tm when screened by the Metcalc software (Göttingen, Germany).

All designed genotype-specific primers were screened for their capability of amplifying the 5' end of an NS5B nucleic acid present in HCV-positive serum samples. Table 16 gives examples of sense and anti-sense primers per genotype that are capable of amplifying the NS5B fragment, according to the further described methodology. The efficiency of the amplification protocols was evaluated using HCV-positive samples from the HCV genotypes 3 to 5 covering a wide range of viral loads.

It was demonstrated that the selected pairs of primers with SEQ ID NO 15 and SEQ ID NO 21 (genotype 3), SEQ ID NO 16 and SEQ ID NO 22 (genotype 4), SEQ ID NO 17 and SEQ ID NO 23 (genotype 5), designed within the two defined NS5B regions, are widely effective for amplification of the targeted NS5B region of the respective HCV genotypes 3 to 5. 23 HCV genotype 3, 5 HCV genotype 4 and 11 HCV genotype 5 positive serum samples were evaluated following a one-step RT-PCR protocol as further described under exactly the same conditions, except changing the set of primers per genotype (see Table 16). The results are summarized in Tables 17 to 19. The expected sizes of NS5B cDNA bands of about 1000 bp were detected in respectively all HCV genotype 3-5 positive serum samples.

Hereto, extraction of RNA from all serum samples was performed with the Qiagen QiAamp MiniElute Virus Spin kit (Venlo, The Netherlands) according to the manufacturar's instructions. Subsequently, RT-PCR was performed to amplify the targeted 5' end NS5B fragment using the Invitrogen SuperScript III One Step RT-PCR System with Platinum Taq DNA polymerase (Merelbeke, Belgium), as further described below. This approach minimizes the possibility for introduction of contaminants into reactions between the RT and PCR steps, since the RT and PCR reactions are carried out sequentially, without opening of reaction tubes between steps. The following one step RT-PCR protocol was developed: PCR was performed from 15 uL of RNA, starting with an RT step consisting of 30 min at 55 °C, followed by 2 min at 94 °C. Next, 40 cycles were performed with each consisting of 30 s at 94 °C, 30 s at 54 °C and 2 min at 68 °C . The extension time for the last cycle was increased to 5 min. The reactions were carried out with 2 µL SuperScript III RT /Platinum Taq polymerase mix in 25 uL 2X reaction mix (containing 200 µM dNTP and 1.6 mM MgCl₂) and 5.5 µL RNAse free water, in the presence of 2.5 µl primer mix (0.5 µM, containing 10 pmol/µL of each primer). The PCR products were electrophoresed in 2% agarose gel and fragments were detected by ethidium bromide staining and UV illumination.

In conclusion, the described methodology provides a standardized and rapid amplification system for the targeted 5' end of an HCV NS5B nucleic acid fragment of any of HCV genotypes 3 to 5. From Table 16 it can moreover be derived that all genotype-specific sense primers locate in the same HCV NS5B region spanning nucleotides 7500 to 7521 and similarly, that all genotype-specific antisense primers locate in the same HCV NS5B region spanning nucleotides 8470 to 8491. As such, these two defined HCV NS5B regions are universal regions wherein any primer according to genotype can be designed capable of amplification of the NS5B fragment.

### EXAMPLE 11. Sequencing of the NS5B amplified product derived from HCV positive serum samples

The designed sense and antisense primers according to HCV genotypes 3 to 5 as described in Example 10 (see Table 16) were succesfully used as sequencing primers to obtain complete coverage of both strands of the resulting amplified NS5B fragment of each genotype. As expected, the nucleic acid sequence of the amplified NS5B fragments derived from the above-mentioned HCV-positive serum samples about 1000 bp was confirmed. The present invention thus provides a uniform and rapid RT-PCR based tool for amplification and sequencing of the 3' end of an NS5B nucleic acid fragment of any one of HCV genotypes 1 to 6.

**Table 16.**

| Genotype | SEQ ID NO | Sense or antisense | Position* | 5'-3' sequence |
|---|---|---|---|---|
| 3 | 15 | sense | 7500-7517 | TCCATGCCKCCTCTYGAG |
| | 21 | Antisense | 8470-8491 | ATGTAACARGTGATTGTGTTGC |
| 4 | 16 | Sense | 7500-7518 | CGTCAATGCCTCCACTAGAG |
| | 22 | Antisense | 8473-8491 | AGRTAGCACGTCAGTGTGT |
| 5 | 17 | Sense | 7500-7517 | TCCATGCCTCCGCTRGAG |
| | 23 | Antisense | 8470-8488 | TAGCACGTCATGGTGTTGC |

| | | | | |
|---|---|---|---|---|
| *The nucleotide positions correspond with the nucleotide numbering of HCV strain HCV-H (GenBank accession no. M67463). Y is T or C ; K is G or T; R is G or A | | | | |

**Table 17**

| Sample | Genotype | Viral Load | NS5B fragment amplified with primer pair SEQ ID NO 15/SEQ ID NO 21 |
|---|---|---|---|
| 248-137 | 3a | 100.599 | ++ |
| IG93851 | 3a | 257.400 | + |
| 235-008 | 3a | 538.866 | ++ |
| 235-014 | 3a | 850.881 | ++ |
| IG93823 | 3a | 925.600 | + |
| IG89658 | 3a | 1.080.000 | +++ |
| IG92572 | 3a | 1.482.000 | ++ |
| INNO3026 | 3 | 1.852.646 | ++ |
| 248-034 | 3a | 1.871.533 | ++ |
| IG93711 | 3a | 3.244.800 | ++ |
| IG93551 | 3 | 5.028.400 | ++ |
| 235-011 | 3a | 15.895.142 | ++ |
| 235-012 | 3a | 27.320.368 | +++ |
| IG57160 | 3 | n.a. | ++ |
| IG57180 | 3a | n.a. | ++ |
| IG57191 | 3 | n.a. | ++ |
| IG57222 | 3 | n.a. | ++ |
| IG57025 | 3a | n.a. | ++ |
| IG94407 | 3 | n.a. | ++ |
| IG94408 | 3 | n.a. | + |
| IG94413 | 3 | n.a. | ++ |
| IG94415 | 3 | n.a. | ++ |
| IG57007 | 3b | n.a. | ++ |

| | | | |
|---|---|---|---|
| +, ++, +++ : amplification product with increasing signal intensity, respectively | | | |

**Table 18**

| Sample | Genotype | Viral Load | NS5B fragment amplified with primer pair SEQ ID NO 16/SEQ ID NO 22 |
|---|---|---|---|
| IG93875 | 4c, 4d | 198.640 | ++ |
| IG93876 | 4c, 4d | 774.800 | ++ |
| IG92577 | 4a | 1.138.800 | ++ |
| INNO3032 | 4 | 3.739.715 | ++ |
| IG94403 | 4 | n.a. | + |

| | | | |
|---|---|---|---|
| +, ++ : amplification product with increasing signal intensity, respectively | | | |

**Table 19**

| Sample | Genotype | Viral Load | NS5B fragment amplified with primer pair SEQ ID NO 17/SEQ ID NO 23 |
|---|---|---|---|
| 235-020 | 5a | 2.204.914 | ++ |
| IG92553 | 5a | 2.332.990 | ++ |
| IG92548 | 5a | 2.600.000 | ++ |
| IG92579 | 5a | 3.525.600 | ++ |
| 235-003 | 5a | 3.988.707 | ++ |
| IG92550 | 5a | 7.061.980 | + |
| IG92554 | 5 | 8.554.286 | + |
| IG92549 | 5a | 10.420.108 | + |
| 235-013 | 5a | 15.852.242 | ++ |
| IG92551 | 5a | n.a. | ++ |
| IG92552 | 5a | n.a. | + |

| | | | |
|---|---|---|---|
| +, ++ : amplification product with increasing signal intensity, respectively | | | |

### EXAMPLE 12. Primers capable of amplifying the 5' end of an NS5B nucleic acid of HCV genotype 6

Sense and antisense primers were designed for amplification of the 5' end of an NS5B nucleic acid of HCV genotype 6 within the HCV NS5B region spanning nucleotides 7500 to 7521 and nucleotides 8529 to 8554 respectively. The nucleotide positions are according to the nucleotide numbering for HCV strain HCV-H (GenBank accession no. M67463). Due to the variability of the genome within a genotype, wobbles were sometimes included in the primer sequence. This was only done for frequently common polymorphisms that gave rise to a too low Tm when screened by the Metcalc software (Göttingen, Germany).

All designed genotype-specific primers were screened for their capability of amplifying the 5' end of an NS5B nucleic acid present in HCV-positive serum samples. Table 20 gives examples of sense and anti-sense primers for genotype 6 that are capable of amplifying the NS5B fragment, according to the further described methodology. The efficiency of the amplification protocols was evaluated using HCV-positive samples of HCV genotype 6 covering a wide range of viral loads.

It was demonstrated that the selected pairs of primers with SEQ ID NO 18 and SEQ ID NO 24, designed within the two defined NS5B regions, are widely effective for amplification of the targeted NS5B region of HCV genotype 6. 11 HCV genotype 6 positive serum samples were evaluated following a one-step RT-PCR protocol as further described under exactly the same conditions. The results are summarized in Table 21. The expected sizes of NS5B cDNA bands of about 1000 bp were detected in all HCV genotype 6 positive serum samples.

Hereto, extraction of RNA from all serum samples was performed with the Qiagen QiAamp MiniElute Virus Spin kit (Venlo, The Netherlands) according to the manufacturar's instructions. Subsequently, RT-PCR was performed to amplify the targeted 5' end NS5B fragment using the Invitrogen Superscript III One Step RT-PCR System with Platinum Taq DNA polymerase (Merelbeke, Belgium), as further described below. This approach minimizes the possibility for introduction of contaminants into reactions between the RT and PCR steps, since the RT and PCR reactions are carried out sequentially, without opening of reaction tubes between steps. The following one step RT-PCR protocol was developed: PCR was performed from 15 uL of RNA, starting with an RT step consisting of 30 min at 55 °C, followed by 2 min at 94 °C. Next, 40 cycles were performed with each consisting of 30 s at 94 °C, 30 s at 54 °C and 2 min at 68 °C . The extension time for the last cycle was increased to 5 min. The reactions were carried out with 2 µL SuperScript III RT /Platinum Taq polymerase mix in 25 uL 2X reaction mix (containing 200 µM dNTP and 1.6 mM MgCl₂) and 5.5 µL RNAse free water, in the presence of 2.5 µl primer mix (0.5 µM, containing 10 pmol/µL of each primer). The PCR products were electrophoresed in 2% agarose gel and fragments were detected by ethidium bromide staining and UV illumination.

In conclusion, the described methodology provides a standardized and rapid amplification system for the targeted 5' end of an HCV NS5B nucleic acid fragment of HCV genotype 6. The two defined HCV NS5B regions are universal regions wherein any primer according to genotype 6 can be designed capable of amplification of the NS5B fragment.

### EXAMPLE 13. Sequencing of the NS5B amplified product derived from HCV genotype 6 positive serum samples

The designed sense and antisense primers according to HCV genotype 6 as described in Example 12 (see Table 20) were succesfully used as sequencing primers to obtain complete coverage of both strands of the resulting amplified NS5B fragment of genotype 6. As expected, the nucleic acid sequence of the amplified NS5B fragments derived from the above-mentioned HCV-positive serum samples of about 1000 bp was confirmed. The present invention thus provides a uniform and rapid RT-PCR based tool for amplification and sequencing of the 5' end of an NS5B nucleic acid fragment of HCV genotype 6.

**Table 20.**

| Genotype | SEQ ID NO | Sense or antisense | Position* | 5'-3' sequence |
|---|---|---|---|---|
| 6 | 18 | Sense | 7500-7517 | TCAATGCCCCCYCTYGAG |
| | 24 | Antisense | 8534-8554 | TCTCCGCAYACCARCATGTC |

| | | | | |
|---|---|---|---|---|
| *The nucleotide positions correspond with the nucleotide numbering of HCV strain HCV-H (GenBank accession no. M67463). Y is T or C ; R is G or A | | | | |

**Table 21**

| Sample | Genotype | Viral Load | NS5B fragment amplified with primer pair SEQ ID NO 18/SEQ ID NO 24 |
|---|---|---|---|
| 242-005 | 6a | 113.646 | + |
| 242-007 | 6a | 529.490 | + |
| 242-044 | 6a | 543.967 | ++ |
| 242-051 | 6a | 629.086 | ++ |
| 242-001 | 6a | 715.380 | ++ |
| 242-010 | 6a | 1.474.840 | ++ |
| 242-012 | 6a | 1.614.475 | ++ |
| 242-052 | 6a | 2.106.031 | ++ |
| 247-004 | 6a | 2.225.990 | ++ |
| 242-016 | 6a | 6.515.876 | ++ |
| IG94414 | 6b | n.a. | ++ |

| | | | |
|---|---|---|---|
| +, ++ : amplification product with increasing signal intensity, respectively | | | |

### REFERENCE LIST

Beaulieu, P. L. and Tsantrizos, Y. S. (2004) Inhibitors of the HCV NS5B polymerase: new hope for the treatment of hepatitis C infections. Curr.Opin.Investig.Drugs. 5, 838-850.
Bukh, J., Miller, R. H. and Purcell, R. H. (1995) Genetic heterogeneity of hepatitis C virus: quasispecies and genotypes. Semin.Liver Dis. 15, 41-63.
Cairns, M. J., King, A. and Sun, L. Q. (2000) Nucleic acid mutation analysis using catalytic DNA. Nucleic Acids Res 28, E9.
Carroll, S. S., Tomassini, J. E., Bosserman, M., Getty, K., Stahlhut, M. W., Eldrup, A. B., Bhat, B., Hall, D., Simcoe, A. L., LaFemina, R., Rutkowski, C. A., Wolanski, B., Yang, Z., Migliaccio, G., De Francesco, R., Kuo, L. C., MacCoss, M. and Olsen, D. B. (2003) Inhibition of hepatitis C virus RNA replication by 2'-modified nucleoside analogs. J Biol Chem 278, 11979-11984.
Cusi, M. G., Valassina, M. and Valensin, P. E. (1994) Comparison of M-MLV reverse transcriptase and Tth polymerase activity in RT-PCR of samples with low virus burden. Biotechniques 17, 1034-1036.
De Francesco, R., Tomei, L., Altamura, S., Summa, V. and Migliaccio, G. (2003) Approaching a new era for hepatitis C virus therapy: inhibitors of the NS3-4A serine protease and the NS5B RNA-dependent RNA polymerase. Antiviral Res 58, 1-16.
Drmanac, R., Drmanac, S., Strezoska, Z., Paunesku, T., Labat, I., Zeremski, M., Snoddy, J., Funkhouser, W. K., Koop, B. and Hood, L. (1993) DNA sequence determination by hybridization: a strategy for efficient large-scale sequencing. Science 260, 1649-1652.
Farci, P., Bukh, J. and Purcell, R. H. (1997) The quasispecies of hepatitis C virus and the host immune response. Springer.Semin.Immunopathol. 19, 5-26.
Grakoui, A., Wychowski, C., Lin, C., Feinstone, S. M. and Rice, C. M. (1993) Expression and identification of hepatitis C virus polyprotein cleavage products. J Virol. 67, 1385-1395.
Griffin, T. J. and Smith, L. M. (2000) Single-nucleotide polymorphism analysis by MALDI-TOF mass spectrometry. Trends. Biotechnol. 18, 77-84.
Hacia, J. G., Brody, L. C., Chee, M. S., Fodor, S. P. and Collins, F. S. (1996) Detection of heterozygous mutations in BRCA1 using high density oligonucleotide arrays and two-colour fluorescence analysis. Nat Genet 14, 441-447.
Healey, B. G., Matson, R. S. and Walt, D. R. (1997) Fiberoptic DNA sensor array capable of detecting point mutations. Anal.Biochem 251, 270-279.
Inchauspe, G., Zebedee, S., Lee, D. H., Sugitani, M., Nasoff, M. and Prince, A. M. (1991) Genomic structure of the human prototype strain H of hepatitis C virus: comparison with American and Japanese isolates. Proc.Natl.Acad.Sci.U.S.A. 88, 10292-10296.
James, W. and al-Shamkhani, A. (1995) RNA enzymes as tools for gene ablation. Curr Opin.Biotechnol. 6, 44-49.
Kenney, M., Ray, S. and Boles, T. C. (1998) Mutation typing using electrophoresis and gel-immobilized Acrydite probes. Biotechniques 25, 516-521.
Kukolj, G., McGibbon, G. A., McKercher, G., Marquis, M., Lefebvre, S., Thauvette, L., Gauthier, J., Goulet, S., Poupart, M. A. and Beaulieu, P. L. (2005) Binding site characterization and resistance to a class of non-nucleoside inhibitors of the hepatitis C virus NS5B polymerase. J Biol Chem 280, 39260-39267.
Kwok, S., Kellogg, D. E., McKinney, N., Spasic, D., Goda, L., Levenson, C. and Sninsky, J. J. (1990) Effects of primer-template mismatches on the polymerase chain reaction: human immunodeficiency virus type 1 model studies. Nucleic.Acids.Res 18, 999-1005.
Lauer, G. M. and Walker, B. D. (2001) Hepatitis C virus infection. N.Engl.J Med 345, 41-52.
Love, R. A., Parge, H. E., Yu, X., Hickey, M. J., Diehl, W., Gao, J., Wriggers, H., Ekker, A., Wang, L., Thomson, J. A., Dragovich, P. S. and Fuhrman, S. A. (2003) Crystallographic identification of a noncompetitive inhibitor binding site on the hepatitis C virus NS5B RNA polymerase enzyme. J Virol. 77, 7575-7581.
Maertens, G. and Stuyver, L. (1997) Genotypes and Genetic Variation of Hepatitis C Virus. In The Molecular Medicine of Viral Hepatitis, ed. T. J. Harrison and A. J. Zuckerman, pp. 183-233. Wiley, John & Sons, Incorporated.
Majzoub, J. A., Rich, A., van Boom, J. and Habener, J. F. (1983) Vasopressin and oxytocin mRNA regulation in the rat assessed by hybridization with synthetic oligonucleotides. J Biol.Chem 258, 14061-14064.
Manns, M. P., McHutchison, J. G., Gordon, S. C., Rustgi, V. K., Shiffman, M., Reindollar, R., Goodman, Z. D., Koury, K., Ling, M. and Albrecht, J. K. (2001) Peginterferon alfa-2b plus ribavirin compared with interferon alfa-2b plus ribavirin for initial treatment of chronic hepatitis C: a randomised trial. Lancet. 358, 958-965.
Mo, H., Lu, L., Pilot-Matias, T., Pithawalla, R., Mondal, R., Masse, S., Dekhtyar, T., Ng, T., Koev, G., Stoll, V., Stewart, K. D., Pratt, J., Donner, P., Rockway, T., Maring, C. and Molla, A. (2005) Mutations conferring resistance to a hepatitis C virus (HCV) RNA-dependent RNA polymerase inhibitor alone or in combination with an HCV serine protease inhibitor in vitro. Antimicrob.Agents.Chemother. 49, 4305-4314.
Myers, T. W. and Gelfand, D. H. (1991) Reverse transcription and DNA amplification by a Thermus thermophilus DNA polymerase. Biochemistry 30, 7661-7666.
Nguyen, T. T., Gates, A. T., Gutshall, L. L., Johnston, V. K., Gu, B., Duffy, K. J. and Sarisky, R. T. (2003) Resistance profile of a hepatitis C virus RNA-dependent RNA polymerase benzothiadiazine inhibitor. Antimicrob.Agents.Chemother. 47, 3525-3530.
Saiki, R. K., Walsh, P. S., Levenson, C. H. and Erlich, H. A. (1989) Genetic analysis of amplified DNA with immobilized sequence-specific oligonucleotide probes. Proc.Natl.Acad.Sci.U.S.A. 86, 6230-6234.
Sapolsky, R. J., Hsie, L., Berno, A., Ghandour, G., Mittmann, M. and Fan, J. B. (1999) High-throughput polymorphism screening and genotyping with high-density oligonucleotide arrays. Genet Anal. 14, 187-192.
Shiffman, M. L. (1999) Improvement in liver histopathology associated with interferon therapy in patients with chronic hepatitis C. Viral Hepatitis, reviews 5, 27-43.
Shimotohno, K., Tanji, Y., Hirowatari, Y., Komoda, Y., Kato, N. and Hijikata, M. (1995) Processing of the hepatitis C virus precursor protein. J Hepatol. 22, 87-92.
Simmonds, P., Alberti, A., Alter, H. J., Bonino, F., Bradley, D. W., Brechot, C., Brouwer, J. T., Chan, S. W., Chayama, K. and Chen, D. S. (1994) A proposed system for the nomenclature of hepatitis C viral genotypes. Hepatology 19, 1321-1324.
Simmonds, P., Bukh, J., Combet, C., Deleage, G., Enomoto, N., Feinstone, S., Halfon, P., Inchauspe, G., Kuiken, C., Maertens, G., Mizokami, M., Murphy, D. G., Okamoto, H., Pawlotsky, J. M., Penin, F., Sablon, E., Shin, I., Stuyver, L. J., Thiel, H. J., Viazov, S., Weiner, A. J. and Widell, A. (2005) Consensus proposals for a unified system of nomenclature of hepatitis C virus genotypes. Hepatology 42, 962-973.
Smith, R. D., Cheng, X., Bruce, J. E., Hofstadler, S. A. and Anderson, G. A. (1994) Trapping detection and reaction of very large single molecular ions by mass spectrometry. Nature 369, 137-139.
Sreevatsan, S., Bookout, J. B., Ringpis, F. M., Pottathil, M. R., Marshall, D. J., de Arruda, M., Murvine, C., Fors, L., Pottathil, R. M. and Barathur, R. R. (1998) Algorithmic approach to high-throughput molecular screening for alpha interferon-resistant genotypes in hepatitis C patients. J Clin Microbiol 36, 1895-1901.
Stuyver, L., Rossau, R. and Maertens, G. (1996a) Line probe assays for the detection of hepatitis B and C virus genotypes. Antivir.Ther 1 Suppl 3, 53-57.
Stuyver, L., Wyseur, A., Rombout, A., Louwagie, J., Scarcez, T., Verhofstede, C., Rimland, D., Schinazi, R. F. and Rossau, R. (1997) Line probe assay for rapid detection of drug-selected mutations in the human immunodeficiency virus type 1 reverse transcriptase gene. Antimicrob Agents Chemother 41, 284-291.
Stuyver, L., Wyseur, A., Van Arnhem, W., Hernandez, F. and Maertens, G. (1996b) Second-generation line probe assay for hepatitis C virus genotyping. J Clin.Microbiol. 34, 2259-2266.
Summa, V., Petrocchi, A., Matassa, V. G., Taliani, M., Laufer, R., De Francesco, R., Altamura, S. and Pace, P. (2004) HCV NS5b RNA-dependent RNA polymerase inhibitors: from alpha,gamma-diketoacids to 4,5-dihydroxypyrimidine- or 3-methyl-5-hydroxypyrimidinonecarboxylic acids. Design and synthesis. J Med Chem 47, 5336-5339.
Tan, S. L., Pause, A., Shi, Y. and Sonenberg, N. (2002) Hepatitis C therapeutics: current status and emerging strategies. Nat Rev Drug Discov 1, 867-881.
Tomei, L., Altamura, S., Bartholomew, L., Biroccio, A., Ceccacci, A., Pacini, L., Narjes, F., Gennari, N., Bisbocci, M., Incitti, I., Orsatti, L., Harper, S., Stansfield, I., Rowley, M., De Francesco, R. and Migliaccio, G. (2003) Mechanism of action and antiviral activity of benzimidazole-based allosteric inhibitors of the hepatitis C virus RNA-dependent RNA polymerase. J Virol. 77, 13225-13231.
Tomei, L., Altamura, S., Bartholomew, L., Bisbocci, M., Bailey, C., Bosserman, M., Cellucci, A., Forte, E., Incitti, I., Orsatti, L., Koch, U., De Francesco, R., Olsen, D. B., Carroll, S. S. and Migliaccio, G. (2004) Characterization of the inhibition of hepatitis C virus RNA replication by nonnucleosides. J Virol. 78, 938-946.
Tomei, L., Altamura, S., Paonessa, G., De Francesco, R. and Migliaccio, G. (2005) HCV antiviral resistance: the impact of in vitro studies on the development of antiviral agents targeting the viral NS5B polymerase. Antivir.Chem Chemother. 16, 225-245.
Walewski, J. L., Keller, T. R., Stump, D. D. and Branch, A. D. (2001) Evidence for a new hepatitis C virus antigen encoded in an overlapping reading frame. RNA. 7, 710-721.
Wang, M., Ng, K. K., Cherney, M. M., Chan, L., Yannopoulos, C. G., Bedard, J., Morin, N., Nguyen-Ba, N., Alaoui-Ismaili, M. H., Bethell, R. C. and James, M. N. (2003) Non-nucleoside analogue inhibitors bind to an allosteric site on HCV NS5B polymerase. Crystal structures and mechanism of inhibition. J Biol Chem 278, 9489-9495.
Whitcombe, D., Theaker, J., Guy, S. P., Brown, T. and Little, S. (1999) Detection of PCR products using self-probing amplicons and fluorescence. Nat Biotechnol. 17, 804-807.
Xu, Z., Choi, J., Yen, T. S., Lu, W., Strohecker, A., Govindarajan, S., Chien, D., Selby, M. J. and Ou, J. (2001) Synthesis of a novel hepatitis C virus protein by ribosomal frameshift. EMBO J20, 3840-3848*.*

## Claims

1. A method for amplification of an HCV NS5B nucleic acid of HCV genotypes 1 to 6 comprising the step of designing a set of primers per genotype in the NS5B genomic region, wherein said set of primers for amplifying said HCV NS5B nucleic acid is **characterized in that** at least one antisense primer per genotype is used that is capable of annealing to the NS5B genomic region spanning nucleotides 8529 to 8546, and wherein said set of primers further comprises a sense primer that is capable of annealing to the NS5B genomic region spanning nucleotides 7536 to 7555 for genotype 1,
wherein the nucleotide positions correspond with the nucleotide numbering for HCV strain HCV-H, and amplifying said HCV NS5B nucleic acids of HCV genotypes 1 to 6 using a simultaneous PCR amplification method with said set of primers.

2. A method for determining the sequence of an HCV NS5B nucleic acid of HCV genotypes 1 to 6 wherein said NS5B nucleic acid is amplified according to the method of claim 1 prior to or concurrent with determining said sequence.

3. A method for evaluating the sequence variations in an HCV NS5B nucleic acid of HCV genotypes 1 to 6 wherein the sequence of said NS5B nucleic acid is determined according to the method of claim 2 prior to evaluating the sequence variations.

4. The method of claim 3 wherein said sequence variations are mutations are chosen from the group consisting of
- mutations that are induced by HCV antiviral agents,
- mutations causing HCV drug resistance, and
- genotype-specific nucleotide variations.

5. The method of any of claims 1 to 4 wherein said amplification is a one-step RT-PCR.

6. The method of claim 1 which is part of a multiplex amplification method.

7. The method of any of claims 1 to 6 wherein the HCV NS5B nucleic acid is present in a biological sample.

8. A set of primers for amplification of an HCV NS5B nucleic acid of HCV genotypes 1 to 6, said set of primers **characterized in that** it comprises at least one antisense primer per genotype that is capable of annealing to the NS5B genomic region spanning nucleotides 8529 to 8546, and said set of primers being further **characterized in that** it comprises a sense primer that is capable of annealing to the NS5B genomic region spanning nucleotides 7536 to 7555 for genotype 1,
wherein the nucleotide positions correspond with the nucleotide numbering for HCV strain HCV-H.

9. Use of a set of primers according to claim 8 for the manufacture of a kit for amplification of an HCV NS5B nucleic acid of HCV genotypes 1 to 6.

10. A kit for amplification of an HCV NS5B nucleic acid of HCV genotypes 1 to 6, said kit comprising a set of primers according to claim 8.

11. The kit according to claim 10 which is a kit for multiplex amplification.


SEQUENCE LISTING

<110> Innogenetics N.V.

<120> Methodology for analysis of sequence variations within the HCV NS5B
genomic region

<130> 183 PCT

<150> US 60/852,983
<151> 2006-10-20

<150> EP 06122668.4
<151> 2006-10-20

<150> EP 06122675.9
<151> 2006-10-20

<150> US 60/852,982
<151> 2006-10-20

<150> US 60/852,984
<151> 2006-10-20

<150> EP 06122670.0
<151> 2006-10-20

<150> EP 06122678.3
<151> 2006-10-20

<150> US 60/853,015
<151> 2006-10-20

<150> US 60/853,016
<151> 2006-10-20

<150>  EP 06122673.4

<151>  2006-10-20

<160>  24

<170>  PatentIn version 3.1

<210>  1

<211>  21

<212>  DNA

<213>  Hepatitis C virus

<400>  1
tgayacccgc tgytttgact c                                                21

<210>  2

<211>  22

<212>  DNA

<213>  Hepatitis C virus

<400>  2
gagacasgct gtgatawatg tc                                               22

<210>  3

<211>  21

<212>  DNA

<213>  Hepatitis C virus

<400>  3
tgayacccgm tgctttgact c                                                21

<210>  4

<211>  23

<212>  DNA

<213>  Hepatitis C virus

<400>  4

cgcgacacgc tgtgataaat gtc 23

<210> 5

<211> 20

<212> DNA

<213> Hepatitis C virus

<400> 5
gacacycgyt gctttgactc 20

<210> 6

<211> 22

<212> DNA

<213> Hepatitis C virus

<400> 6
gygacacgct gtgataaatg tc 22

<210> 7

<211> 21

<212> DNA

<213> Hepatitis C virus

<400> 7
tgatacccgc tgctttgact c 21

<210> 8

<211> 22

<212> DNA

<213> Hepatitis C virus

<400> 8
gagacatgct gtgataaatg tc 22

<210> 9

<211> 21

<212> DNA

<213> Hepatitis C virus

<400>  9
tgayacccgc tgctttgact c                                                21

<210>  10
<211>  23
<212>  DNA
<213>  Hepatitis C virus

<400>  10
cgggacatgc tgtgataaat gtc                                              23

<210>  11
<211>  20
<212>  DNA
<213>  Hepatitis C virus

<400>  11
gacaccagrt gytttgattc                                                  20

<210>  12
<211>  21
<212>  DNA
<213>  Hepatitis C virus

<400>  12
ggacacgctg tgataaatgt c                                                21

<210>  13
<211>  20
<212>  DNA
<213>  Hepatitis C virus

<400>  13
gatctcagyg acgggtcwtg                                                  20

<210>  14
<211>  22
<212>  DNA
<213>  Hepatitis C virus

<400> 14
gctccatgtc atactcctgg ac 22

<210> 15
<211> 18
<212> DNA
<213> Hepatitis C virus

<400> 15
tccatgcckc ctctygag 18

<210> 16
<211> 20
<212> DNA
<213> Hepatitis C virus

<400> 16
cgtcaatgcc tccactagag 20

<210> 17
<211> 18
<212> DNA
<213> Hepatitis C virus

<400> 17
tccatgcctc cgctrgag 18

<210> 18
<211> 18
<212> DNA
<213> Hepatitis C virus

<400> 18
tcaatgcccc cyctygag 18

<210> 19
<211> 18
<212> DNA
<213> Hepatitis C virus

<400>  19
cacgagcats gtgcagtc                                                    18

<210>  20
<211>  19
<212>  DNA
<213>  Hepatitis C virus

<400>  20
tacctggtca tagcctccg                                                   19

<210>  21
<211>  22
<212>  DNA
<213>  Hepatitis C virus

<400>  21
atgtaacarg tgattgtgtt gc                                               22

<210>  22
<211>  19
<212>  DNA
<213>  Hepatitis C virus

<400>  22
agrtagcacg tcagtgtgt                                                   19

<210>  23
<211>  19
<212>  DNA
<213>  Hepatitis C virus

<400>  23
tagcacgtca tggtgttgc                                                   19

<210>  24
<211>  20
<212>  DNA
<213>  Hepatitis C virus

<400>  24
tctccgcaya ccarcatgtc                                                   20